# EUROPEAN PATENT APPLICATION

(11) **EP 3 266 440 A1**
(43) Date of publication of application: **10.01.2018**
(21) Application number: 15830753.8
(22) Date of filing: 18.06.2015
(51) Int. Cl.: A61K 8/02

(54) **MULTI-PURPOSE SELF-TEMPERATURE CONTROL FILM AND FACE MASK AND EYE MASK MANUFACTURED THEREFROM**

(30) Priority: 06.08.2014 CN 201410381963; 06.08.2014 CN 201410382344; 06.08.2014 CN 201410381992; 06.08.2014 CN 201410381980
(71) Applicant: Handy Technology (Zhuhai) Ltd., Zhuhai, Guangdong 519060 (CN)
(72) Inventor: HUANG, Yingluo, ZhuHai Guangdong 519099 (CN); CHEN, Lei, ZhuHai Guangdong 519099 (CN)
(74) Representative: Meyer, Thorsten
(86) International application number: PCT/CN2015/081721
(87) International publication number: WO 2016/019764

(57) **Abstract**

The present invention discloses a multifunctional temperature self-regulating multilayer film, from outside to inside comprising at least: a carrier layer (1) loaded with functional material, and a thermal-insulation layer (2) directly contacted with the skin (4); wherein the carrier layer (1) loaded with functional material has a sealed cavity sealed with a flexible water-impermeable material, the sealed cavity is at least loaded with a refrigerating or heat-generating chemical material (11); wherein thermal-insulation layer (2) is made of a material having waterproof and thermal-insulation properties. The present multilayer film can be manufactured into a face mask or eye mask for use on facial skin. If the multilayer film is loaded with heat-generating chemical material, the thermal-insulation layer (2) ensures that the heat given off from the carrier layer (1) is uniformly and stably transferred to the skin (4), and the heat would not spread out quickly from the skin (4), so as to avoid scalding caused by too high initial temperature and maintain stable and suitable holding temperature. If the multilayer film is loaded with refrigerating chemical material, the thermal-insulation layer (2) prevents the carrier layer (1) from instantaneously absorbing the heat of the skin, so as to avoid local frostbite and perform uniform heat exchange between the carrier layer (1) and the skin (4), and significantly prolong the duration of refrigerating.

## Description

### FIELD OF THE INVENTION

The present invention relates to a multifunctional temperature self-regulating multilayer film, and particularly relates to a multilayer film which has a refrigerating or heat-generating function, or both the refrigerating and heat-generating functions, and more particularly relates to a three-dimensional face mask, a face mask, or an eye mask made of the multifunctional temperature self-regulating multilayer film.

### BACKGROUND OF THE INVENTION

Hot compress causes the blood vessels to dilate and increases the blood flow in that area, so as to accelerate the metabolism in that area and help to heal disease. In addition, hot compress can also relieve muscle spasm and help to eliminate extravasated blood and inflammation. Furthermore, hot compress can accelerate the topical absorption of the external medicine in the lesion area, so as to make treatments more effective.

Cold compress causes the blood vessels to shrink and reduces topical congestion, and also induces an inhibitory effect on the sensory nerve, thereby decreasing the pain signals being transmitted to the brain. So, cold compress has an effect on cooling the body, preventing the bleeding, alleviating pain and reducing the swelling.

If a hot compress is performed prior to a cold compress, the blood vessels in that area would dilate under high temperature, then shrink under low temperature, so that the blood flow in that area is further increased. Increased blood flow can provide the lesion area with additional oxygen and nutrients, and help to exclude the metabolic waste, so as to accelerate the metabolism and speeding the process of treatment of diseases in the lesion area.

The existing self-heating chemical pads have several shortcomings as follows: 1) the initial temperature is too high, which would cause low temperature scalding and damage the body tissue; 2) it is inhomogeneous in heating, so as to result in temperature of local part too high; 2) the heat loss is too fast, so as to shorten the duration of heating. The existing self-cooling chemical pads have several shortcomings as follows: 1) the duration of refrigerating is short; 2) it is inhomogeneous in cooling, which would result in topical frostbite; 3) the condensed water would aggregate, so as to result in the pads being wetted with water and difficult to be attached to the body part to be cooled. More importantly, the existing self-heating/ self-cooling chemical pads can't self-regulate temperature, so it can't satisfy the different needs of customers for temperature.

In addition, the products which are capable of performing a hot compress prior to a cold compress are complicated in structure, costly, fussy in operation. For example, external power source or semiconductor wafer is needed, just as disclosed in CN 00252623.9 and CN 02159757.X.

A face mask is a carrier of cosmetics or skincare products, which is usually applied to the face for 15-30 minutes. When the cosmetics or skincare products have been absorbed by the skin, the face mask is taken off from the face. The most essential and important function of the face mask is to clean the skin, since it is not enough to clean the skin just by removing the make-up and washing the face. The face mask also has other functions, such as, skin moisturizing, skin whitening, or skin anti-aging.

Traditional face mask is usually a sheet-mask, which is covered on the face after unfolding. During the contact process, the sheet-mask can temporarily prevent outside air and pollutants from reaching the face, enlarge the skin pores, and accelerate blood circulation and metabolism, so that the metabolites produced by the epidermis cells and accumulated oils can be excluded from the skin. In addition, the water contained in the face mask can permeate into the stratum corneum of the skin, so as to soften and clean the skin. The function of the traditional face mask is single, without the effect of hot compress and/ or cold compress.

The newly designed three-dimensional face mask includes a right side sheet and a left side sheet which are mutually symmetrical along the median line of the face, so that the three -dimensional face mask can be reliably applied to the user's face which has a concavo-convex shape. Compared with the sheet-mask, the three-dimensional face mask can improve the adherence over the user's face, and does not peel off easily. However, the function of the three -dimensional face mask is still single, without the effect of hot compress and/ or cold compress.

An eye mask, as a popular cosmetic product, is usually applied to the eyes, and performs an effective skin care around the eyes. The eye mask is capable of supplying the eyes with moisture and nutrition, relieving fatigue of eyes, eliminating dropsy and dark circles phenomenon. To some extent, the eye mask can also be used to reduce the eye wrinkle caused by aging. With the popularity of electronic products in our daily life, people usually stare at the electronic screen for long hours, which strains the eyes and leads to eyes discomfort, fatigue or dry. When people lack sleeps or have irregular sleep pattern, the damage to eyes is more serious. Therefore, eye care becomes more and more important.

Traditional eye mask is formed by mashing or boiling down the items which have eye care or cleaning function (such as, loofah, honey, tremella) and then applying the puree to the eyes. Commercial eye mask is a sheet-mask which is usually impregnated with an essence, such as, pentapeptide, sodium hyaluronate, vitamin E, vitamin B3, mineral substances or some natural ingredients, so as to achieve the effect of beauty and healthcare. However, the function of the eye mask is still single, without the effect of hot compress and/ or cold compress.

### SUMMARY OF THE INVENTION

The aim of the present invention is to overcome the shortcomings in the prior art and provide a multifunctional temperature self-regulating film, throughout which the energy is distributed evenly, and which can effectively prolong heating time and/or cooling time and reduce peak heating temperature and/or cooling temperature, and can be used in combination with a skincare product, topically-applied medicine or healthcare product. The specific technical solution is as follows: a multifunctional temperature self-regulating film, from outside to inside comprising at least: a carrier layer loaded with functional material, and a thermal-insulation layer directly contacted with the skin; wherein the carrier layer loaded with functional material has a sealed cavity sealed with a flexible water-impermeable material, the sealed cavity is at least loaded with a refrigerating or heat-generating chemical material; wherein the thermal-insulation layer is made of a material having waterproof and thermal-insulation properties.

Compared with the existing products, the present multilayer film comprises an additional thermal-insulation layer formed of a material having waterproof and thermal-insulation properties. When used, if the multilayer film is loaded with a heat-generating chemical material, the thermal-insulation layer ensures the heat given off from the heat-generating layer is evenly and stably transferred to the skin, and the heat would not spread out quickly from the skin, so as to avoid scalding caused by too high initial temperature and maintain stable and suitable holding temperature; if the multilayer film is loaded with refrigerating chemical material, the thermal-insulation layer prevents the refrigerating chemical material from instantly absorbing the energy through the skin, so as to avoid topical frostbite, and the thermal-insulation layer ensures that the energy exchanging between the skin and the carrier layer can be conducted evenly, so as to maintain stable and suitable holding temperature and prolong cooling time. The present multifunctional temperature self-regulating film is not limited to a structure of the above two layers, it may also comprise a package layer or adhesive layer etc. as needed.

Preferably, the thermal-insulation layer is made of metallized polyethylene terephthalate (MPET) film. In an example, the thermal-insulation layer is formed by a physical vapor deposition process, during which the PET film is coated with a layer of metal. The metal is heated and vaporized under vacuum, then the vaporized metal condenses or deposits on the cold PET film which is near the metal vapor source. The color of the MPET film is usually golden or silvery. Of course, other colors may also be possible. In an example, aluminum is used for deposition or condensation, but other metals such as nickel or chromium or metal mixture can also be used. The MPET is much thinner than a metal foil, with a thickness equal to or less than 0.5µm. The MPET film has good flexibility and waterproofhess, closely fits the skin, and will not fade or discolor over time. More importantly, the MPET film has excellent thermal-insulation property, and the addition of metal helps the energy exchanging between the skin and the carrier layer conduct evenly and stably, so as to effectively adjust skin temperature. In addition, golden or silvery surface can suit the user's aesthetic needs. Besides polyethylene terephthalate (PET), oriented polypropylene, nylon, polyethylene and cast polypropylene film can also be used.

Preferably, the flexible water-impermeable material is selected from plastics, synthetic rubbers, synthetic fibers, waterproof fabrics or waterproof papers. There is no limitation to the flexible water-impermeable material, as long as it is waterproof and can be sealed. Preferably, the flexible water-impermeable material is transparent, so that the user can observe the state and color of the refrigerating or heat-generating chemical material. More preferably, the flexible water-impermeable material is transparent PVC.

In one embodiment, the sealed cavity is loaded with a heat-generating chemical material which generates heat when the material changes from liquid to solid, and an actuating means which is used to trigger an exothermic state of the phase change material. The solid-liquid phase change material is a substance which is capable of storing and releasing energy under solid-liquid phase transition. The solid-liquid phase-change material has high energy density and high efficiency, and can absorb energy and release energy at an almost constant temperature. In practice, the solid-liquid phase change material includes organics (such as, paraffins, fatty acids and esters, etc.) and inorganics (such as, salt hydrates, molten salts, metals, etc.), wherein the most widely used is the salt hydrates. The salt hydrates are an important phase change material regarding medium and low temperature, which comprise almost 70 kinds of substances and are available in a melting temperature range from several up to more than one hundred degrees centigrade. In the present invention, it is proposed to select suitable inorganic phase change material according to desired temperature and application. In specific embodiments, alkali and alkaline earth metal halide, sulfate, nitrate, phosphate, carbonate and acetate, etc. could be selected according to different required temperature. The mechanism of using inorganic phase change material to store energy is as follows: when the salt hydrate is heated to its melting temperature, the salt hydrate will absorb heat and dehydrate, so as to transform to anhydrous salt (or another type of salt hydrate with less mole of water), wherein part of salt will dissolve in the dehydrated water to form saturated solution; when the phase-balance is broke, the reverse phase change will occur, that is, the salt will recombine with water to form salt hydrate crystals, during this process, and the absorbed heat will be released. In specific embodiments, the present multilayer film just uses the salt hydrate dehydration and hydration reaction to store and release heat. The actuating means is used to trigger the change of state of the phase change material, such as, a foreign matter or a spring which can strongly vibrate, and there is no special limitation to the actuating means. The actuating means can break the phase-balance of the phage change material around it to form a nucleation center, so that the salt recombines with water to form salt hydrate crystals.

Preferably, after the liquid-to-solid phase change process of the solid-liquid phase change material has been triggered, the multilayer film maintains a heating temperature at 40-55°C, and the heating time lasts 5-30 minutes. Generally speaking, the carrier layer loaded with functional material is configured to heat the skin to a desired and effective temperature, without causing any discomfort. For example, the present film can raise the temperature of the skin to 38-55°C, and the holding time depends upon the specific application, such as longer than 15 minutes, 30 minutes, or even 1 hour. The heating temperature and heating time can be controlled by selecting suitable phase change material and adjusting the loading amount.

Preferably, the solid-liquid phase change material is an oversaturated aqueous solution of sodium acetate (CH₂COONa), and the actuating means is a flexible metal disc which takes a shape of an arc and has a plurality of corrugations on its surface, so as to be easily bent, and the flexible metal disc is located in the oversaturated aqueous solution of sodium acetate. Sodium acetate trihydrate is one of the inorganic phase change materials (salt hydrates) regarding low temperature, which has a melting point of 58 °C, a high latent heat of fusion of 264 J/g, and excellent thermal conductance, and is non-toxic, low-cost, and easily available. The flexible metal disc would vibrate when pressing or bending it by hand, so as to break the phase balance of the oversaturated aqueous solution of sodium acetate, thereby the sodium acetate would form crystals and release heat , and the heating temperature would reach about 40-55°C. The present film can be reused when immersing it into hot water at about 80°C for 3-5 minutes to completely dissolve the sodium acetate crystals and then pressing the flexible metal disc to make the oversaturated aqueous solution of sodium acetate form crystals and release heat.

Preferably, the oversaturated aqueous solution of sodium acetate is added with nucleating agent. The sodium acetate is prone to supercooling and phase separation during the phase change process. In the present invention, "supercooling" refers to the phenomenon that after the film has been reused for many times, the actuating means can't trigger the liquid sodium acetate to crystalize, and the liquid phase can be maintained all the way down to the temperature at which crystal homogeneous nucleation occurs, so that the sodium acetate cannot release latent heat stored. In the present invention, "phase separation "refers to the phenomenon that after the film has been reused for many times, the sodium acetate will precipitate and can't be combined with crystal water, and separation will occur, which will greatly decrease the heat storage capacity of sodium acetate and shorten its period of use. When the nucleating agent is added, the supercooling and phase separation can be effectively inhibited, so the heating stability of the film can be improved and the number of reuse can be increased. Preferably, the nucleating agent is selected from one or more of the group consisting of sodium borate, sodium sulfate, sodium silicate, sodium pyrophosphate, sodium phosphate dibasic dodecahydrate.

Preferably, at least one side of the carrier layer is provided with a fluid reservoir. The fluid reservoir and the carrier layer are manufactured in one piece, and they are interconnected by the existing one-way valve. The fluid reservoir is loaded with fluid, when the fluid reservoirs is pressed by an external force, the fluid loaded in the fluid reservoir is injected into the carrier layer through the one-way valve to adjust the temperature of the carrier layer. The one-way valve ensures that the fluid can only flow to the carrier layer from the fluid reservoir and can't flow to the fluid reservoir from the carrier layer. In practice, different consumers have different needs for the temperature, and the existing hot pack in the absence of external heat sources is difficult to adjust the temperature. In the present invention, when the user feels the temperature of the carrier layer is too high and wants to lower the temperature, he can press the fluid reservoir to make part or all of the fluid, such as clean water, loaded in the fluid reservoir flow to the carrier layer and mix with the solid-liquid phase change heat-generating material, so as to generate an endothermic reaction which causes the temperature of the carrier layer to drop to the desired temperature. Alternatively, after a hot compress has been performed (that is, after the temperature of the carrier layer has dropt to room temperature), the user wants a cold compress, he only needs to perform the operation as described above to cool the carrier layer loaded with functional material. In a specific embodiment, the cooling effect varies depending upon the solid-liquid phase change heat-generating material and/ or the fluid loaded in the fluid reservoir and the loading amount thereof, which can be adjusted according to actual needs, and the final temperature may reach 9-20 °C, the holding time lasts 5-30 minutes. That is, the present multilayer film has both refrigerating and heat-generating functions.

In a specific embodiment, the carrier layer is loaded with a solid refrigerating chemical material, which absorbs heat when the material changes from solid to liquid. At least one side of the carrier layer is provided with a fluid reservoir. The fluid reservoir and the carrier layer are manufactured in one piece, and they are interconnected by the existing one-way valve. The fluid reservoir is loaded with fluid, when the fluid reservoir is pressed by an external force, the fluid loaded in the fluid reservoir is injected into the carrier layer through the one-way valve to dissolve the solid refrigerating chemical material, which would absorb energy. That is to say, the carrier layer and the fluid reservoir are respectively loaded with two materials, and when the two materials are mixed, it will cause an endothermic reaction to achieve refrigerating effect. For example, the solid refrigerating chemical material loaded in the carrier layer is sodium thiosulfate, and the fluid loaded in the fluid reservoir is ammonium nitrate. Press the fluid reservoir and get the two materials mixed, an endothermic reaction will occur. In addition, the user can manually adjust the amount of the fluid entering into the carrier layer by the one-way valve, so as to control temperature as needed.

Preferably, after the solid-to-liquid phase change process of the solid refrigerating chemical material has been triggered, the multilayer film maintains a cooling temperature at -3-20°C, and the cooling time lasts 5-30 minutes. Generally speaking, the carrier layer loaded with functional material is configured to cool the skin to a desired and effective temperature, without causing any discomfort, and the holding time depends upon the specific application, such as longer than 15 minutes, 30minutes, or even 1 hour. The cooling temperature and cooling time can be controlled by selecting suitable phase change material and adjusting the loading amount.

Preferably, the solid refrigerating chemical material which absorbs heat when the material changes from solid to liquid is urea, ammonium chloride, ammonium nitrate or mixture thereof, and urea is more preferable. The fluid loaded in the fluid reservoir is clean water. The weight ratio of the solid refrigerating chemical material to the clean water is 1:1. The solid refrigerating chemical materials mentioned above are commonly used agricultural fertilizers, which are low cost, readily available and environmentally friendly, and have excellent refrigerating effect.

Preferably, a suitable amount of thickener is added in the fluid loaded in the fluid reservoir. The fluid loaded in the fluid reservoir, such as clean water, injected into the carrier layer may accumulate at the bottom of the film due to gravity, however, the thickener prevents the fluid from accumulating at the bottom of the film, that is, the fluid distributes evenly in the carrier layer, so the endothermic or exothermic reaction and the temperature distribution are more homogeneous, which can enhance the comfort of users using it. The Thickener includes natural thickener, such as starch, xanthan, gelatin, guar gum, natural rubber, and agar; fibrous thickener, such as methyl cellulose, hydroxyethyl cellulose, hydroxypropyl methyl cellulose, sodium carboxymethyl cellulose; inorganic thickener, such as bentonite, diatomite, white carbon black, sodium bentonite, silica gel; polymer thickener, such as polyurethane, sodium polyacrylate, polyvinyl alcohol, polyacrylamide, polyvinyl pyrrolidone, poly ethylene oxide, carbomer resin, polyacrylic acid, acrylate copolymer emulsion etc.

Preferably, the carrier layer and/or the fluid reservoir is further loaded with a pigment. For example, an edible pigment with warm color (such as red, pink, orange or yellow) can be added in the oversaturated aqueous solution of sodium acetate loaded in the transparent carrier layer, and a pigment with cold color (such as blue or purple) can be added in the fluid loaded in the fluid reservoir, so the user would see warm color during a hot compress process, and when the clean water loaded in the fluid reservoir is injected into the carrier layer, the warm color is changed by the pigment with cold color. With a reasonable color assortment, the user can estimate the temperature according to the color of the film and can further understand the functions of the present film.

In specific embodiments, the present multifunctional temperature self-regulating multilayer film is ergonomically designed in shape to be applied to various body parts, such as neck, knee, legs, back, waist or hand, to be warmed or cooled. There is no limitation to the shape of the present multilayer film, and it may be circular, oval, rectangular, annular, heart-shaped, triquetrous, or cartoon-shaped, etc., as long as it can match the body part to be applied to. The film can be adhered or attached to the user's body part by traditional chemical methods (such as, using an adhesive) or physical methods (such as, using a strap, velcro or fastener).

Preferably, in order to adhere the present film to the user's body part, there is provided a layer of water-soluble hydrogel on the surface of the thermal-insulation layer that is in contact with the skin. Soluble hydrogel can be used as a body adhesive to adhere the film to the body part. In soluble hydrogel, water is the dispersion medium. When soluble hydrogel is adhered to the skin, it turns from a solid to a liquid state due to body temperature, and permeates into the skin. Therefore, preferably, there is provided some active ingredients in the hydrogel matrix, such as, collagen, hyaluronic acid, arbutin, niacinamide, aromatic essential oil, or other healthcare products, topically-applied medicine etc. Since the present multifunctional temperature self-regulating film comprises an additional thermal-insulation layer formed of a material having waterproof and thermal-insulation properties, the water in the hydrogel can't permeate into the heat-generating layer, so as to avoid affecting peak heating/cooling temperature and heating/cooling effect. Furthermore, hydrogel is helpful to prevent heat from quickly spreading out from the skin.

In one embodiment, the multifunctional temperature self-regulating multilayer film is made into a mask applied to the user's face. Both two sides of the carrier layer are provided with a fluid reservoir, which is made into a long and narrow strip. Either of the fluid reservoirs is provided with a fixture to attach the face mask to the user's head. The present multifunctional temperature self-regulating film not only can be used for a hot compress or a cold compress, but also can be used for alternate hot and hold compress, so as to meet different needs of users. Especially, the alternate hot and cold compress is very effective for skin care. The alternate hot and hold compress can shrink skin pores, accelerate blood circulation, fully clean the dirt in the pores, so as to tender and lighten the skin. It has been demonstrated that repeated about 40°C skin temperature can help to produce HSP protein, repair skin cells, so as to enhance skin elasticity and prevent skin wrinkle. In the present invention, a hot compress at a temperature of about 40°C can help to enlarge skin pores, fully clean the dirt, and absorb the nutrient, and the following cold compress can help to instantly shrink skin pores and maintain the moisture and nutrient, so as to tender and lighten the skin. With an alternate hot and hold compress, the skin would look soften and lighten even without a make-up, and it can also help to repair damaged cells and prevent the formation of wrinkle and spot.

Preferably, the mask is 2-15mm thick. Within such a thickness range, not only the above structure of two layers and the above heating/cooling temperature and time can be achieved, but also the face mask fits tightly to the face. The face mask is routine in size.

The present invention also provides a method of using the multifunctional temperature self-regulating multilayer film. In some cases, the film not only can be used to warm/cool body, but also can be used to accelerate the absorption of skincare or healthcare products. In one embodiment, a skincare product, topically-applied medicine, healthcare product or essential oil is applied to the body prior to applying the present film by a fixture.

The present invention also provides a method of using the multifunctional temperature self-regulating face mask. A skincare product or traditional nourishing mark is applied to the face prior to applying the face mask. Since the present face mask can enlarge facial pores and accelerate the excretion of dirt and sebaceous secretion, the absorption of the effective ingredients in the skincare products is improved, the amount of skincare product used is reduced and skincare effect is enhanced.

Although the present multifunctional temperature self-regulating multilayer film is structurally simple, it has the functions of heating, cooling and alternate heating and cooling. In addition, it is not constrained by environmental resources, does not require energy supply from an external energy source, is safe and stable, uniformly generates heat or absorbs heat, and can effectively prolong heating/cooling time. Especially, the design of the fluid reservoir can meet different needs for temperature. The existing self-heating/ self-cooling chemical pads only have single function, and either it is difficult to adjust the temperature, or the external energy is needed to adjust temperature and the operation is cumbersome. However, the present film has overcome the above shortcomings of the existing self-heating/self-cooling chemical pads. Furthermore, the present multifunctional temperature self-regulating multilayer film is low cost, and the used refrigerating or heat-generating chemical material will not cause a toxin pollution, and can be recycled and used as materials improving the quality of the earth.

On the other hand, the present application provides a multifunctional three-dimensional face mask which can generate heat and absorb heat thereby to perform a hot compress and a cold compress, and can effectively prolong heating time and/or cooling time and reduce peak heating temperature and/or cooling temperature, and throughout which the energy is distributed evenly. The specific technical solution is as follows: a multifunctional temperature self-regulating three-dimensional face mask, from outside to inside comprising at least: a carrier layer loaded with functional material, and a thermal-insulation layer directly contacted with the skin; wherein the carrier layer loaded with functional material has a sealed cavity sealed with a flexible water-impermeable material, the sealed cavity is at least loaded with a refrigerating or heat-generating chemical material; wherein the thermal-insulation layer is made of a material having waterproof and thermal-insulation properties. The present multifunctional temperature self-regulating three-dimensional face mask is not limited to a structure of the above two layers, it may also comprise a package layer or adhesive layer etc. as needed.

Preferably, the thermal-insulation layer is made of metallized polyethylene terephthalate (MPET) film. Preferably, the flexible water-impermeable material is selected from plastics, synthetic rubbers, synthetic fibers, waterproof fabrics or waterproof papers.

In one embodiment, the sealed cavity of the three-dimensional face mask is loaded with a heat-generating chemical material which generates heat when the material changes from liquid to solid, and an actuating means which is used to trigger an exothermic state of the phase change material. According to the disclosure described above, the present three-dimensional face mask is capable of generating heat. Preferably, after the liquid-to-solid phase change process of the solid-liquid phase change material has been triggered, the multilayer three-dimensional face mask maintains a heating temperature at 40-55°C, and the heating time lasts 5-30 minutes.

Preferably, the solid-liquid phase change material is an oversaturated aqueous solution of sodium acetate, and the actuating means is a flexible metal disc which takes a shape of an arc and has a plurality of corrugations on its surface, so as to be easily bent, and the flexible metal disc is located in the oversaturated aqueous solution of sodium acetate. Preferably, the oversaturated aqueous solution of sodium acetate is added with nucleating agent. Preferably, the nucleating agent is selected from one or more of the group consisting of sodium borate, sodium sulfate, sodium silicate, sodium pyrophosphate, sodium phosphate dibasic dodecahydrate.

Preferably, at least one side of the carrier layer is provided with a fluid reservoir. The fluid reservoir and the carrier layer are manufactured in one piece, and they are interconnected by the existing one-way valve. The fluid reservoir is loaded with fluid, when the fluid reservoirs is pressed by an external force, the fluid loaded in the fluid reservoir is injected into the carrier layer through the one-way valve to adjust the temperature of the carrier layer. According to the disclosure described above, the present three-dimensional face mask has both refrigerating and heat-generating functions.

In a specific embodiment, the carrier layer is loaded with solid refrigerating chemical material, which absorbs heat when the material changes from solid to liquid. At least one side of the carrier layer is provided with a fluid reservoir. The fluid reservoir and the carrier layer are manufactured in one piece, and they are interconnected by the existing one-way valve. The fluid reservoir is loaded with fluid, when the fluid reservoir is pressed by an external force, the fluid loaded in the fluid reservoir is injected into the carrier layer through the one-way valve to dissolve the solid refrigerating chemical material, which would absorb energy. According to the disclosure described above, the present three-dimensional face mask has refrigerating function.

Preferably, after the solid-to-liquid phase change process of the solid refrigerating chemical material has been triggered, the three-dimensional face mask maintains a cooling temperature at -3-20°C, and the cooling time lasts 5-30 minutes. Preferably, the solid refrigerating chemical material which absorbs heat when the material changes from solid to liquid is urea, ammonium chloride, ammonium nitrate or mixture thereof, and urea is more preferable. The fluid loaded in the fluid reservoir is clean water. The weight ratio of the solid refrigerating chemical material to the clean water is 1:1.

Preferably, a suitable amount of thickener is added in the fluid loaded in the fluid reservoir of the three-dimensional face mask. Preferably, the carrier layer and/or the fluid reservoir is further loaded with a pigment.

Preferably, the three-dimensional mask is 2-15mm thick. Preferably, in order to adhere the present three-dimensional mask to the face, there is provided a layer of water-soluble hydrogel on the surface of the thermal-insulation layer that is in contact with the skin.

In one embodiment, the three-dimensional face mask comprises a left side sheet and a right side sheet respectively matching the left area of the face and the right area of the face, front edge portions of the left side sheet and the right side sheet are provided with an adhesive material, the left side sheet and the right side sheet are thereby adhered together by the adhesive material along a median line of the face to form a facial concave-convex shape. Generally speaking, the left side sheet and the right side sheet are symmetrical along the median line of the face. Due to this, it is possible to keep this three-dimensional face mask completely adhering to every facial curve, so as to overcome the deficiencies of the traditional face mask, which is built from a single mask sheet and is liable to wrinkle and slack, thereby the traditional one-piece face mask cannot be uniformly and reliably applied to the face and affects the cosmetic and cleaning effect.

Preferably, either of the front edge portions of the left side sheet and the right side sheet has a protrusion projecting outwards from above down and matching the shape of the left area or right area of the external nose, a mouth portion which is a notch matching the mouth is provided below the protrusion, a portion of the front edge portion below the notch has an arc shape with a certain radian and extends downwards to the bottom the face mask; except the notch matching the mouth, either of the front edge portions of the left side sheet and the right side sheet is provided with an adhesive material, and the left side sheet and the right side sheet are thereby adhered together by the adhesive material. Because either of the front edge portions of the left side sheet and the right side sheet has a protrusion projecting outwards from above down and matching the shape of the left area or right area of the external nose, after the left side sheet and the right side sheet are adhered together, the three-dimensional face mask would naturally have a nose-like protrusion from above down on the user's nose, so as to reliably fit the nose. Because a mouth portion which is a notch matching the mouth is provided below the protrusion, and because a portion of the front edge portion below the notch has an arc shape with a certain radian and extends downwards to the bottom the face mask,, after the left side sheet and the right side sheet are adhered together, the lower half of face mask has a curved surface which can reliably fit the jaw, so as to improve the adherence to the face.

Preferably, the three-dimensional face mask according to the present invention is inclosed except that there is provided a hole corresponding to the user's mouth. Preferably, a glabella portion of either of the front edge portions of the left side sheet and the right side sheet which faces a space between the user's eyebrows, just above the protrusion, is formed as an arc which is slightly concaved inwards. The glabella portion is provided with an adhesive material. Due to this, after the left side sheet and the right side sheet are adhered together, the three-dimensional face mask would have a slight concave on a glabella-and- nasion portion, such that the three-dimensional face mask reliably fits the concave on the user's glabella-and-nasion portion. The three-dimensional face mask of this embodiment can clean and rejuvenate most areas of the face skin expect the mouth and nose. In addition, the heat-generating layer and thermal-insulation layer of the three-dimensional face mask can warm and nourish the user's eyes, and thus preventing dry eye syndrome.

Preferably, the three-dimensional face mask according to the present invention is provided with three holes respectively corresponding to the user's mouth and two eyes, that is, the user's mouth and eyes are not covered during use. An eye-and-glabella portion of either of the front edge portions of the left side sheet and the right side sheet which faces the user's eyes and glabella, just above his/her nose, is formed as a notch which is concaved inwards. The notch can be almost semielliptical or semicircular, as long as the eyes are uncovered but the skin around the eyes is almost covered. On either of the front edge portions of the left side sheet and the right side sheet, neither the mouth portion nor the eye-and-glabella portion is provided with adhesive material. That is, the left side sheet and the right side sheet are provided with an adhesive material on either of the front edge portions, except the portions corresponding to the user's mouth and eyes, and the left side sheet and the right side sheet are thereby adhered together by the adhesive material.

Preferably, there is a fixing strap provided on either of the rear edge portions of the left side sheet and the right side sheet, and there is a velcro provided at the end of the fixing strap.

The present multifunctional three-dimensional face mask not only can be used for a hot compress or a cold compress, but also can be used for alternate hot and hold compress, so as to meet different needs of users. Especially, the alternate hot and cold compress is very effective for skin care. The alternate hot and hold compress can shrink skin pores, accelerate blood circulation, fully clean the dirt in the pores, so as to tender and lighten the skin. It has been demonstrated that repeated about 40°C skin temperature can help to produce HSP protein, repair skin cells, so as to enhance skin elasticity and prevent skin wrinkle. In the present invention, a hot compress at a temperature of about 40°C can help to enlarge skin pores, fully clean the dirt, and absorb the nutrient, and the following cold compress can help to instantly shrink skin pores and maintain the moisture and nutrient, so as to tender and lighten the skin. With an alternate hot and hold compress, the skin would look soften and lighten even without a make-up, and it can also help to repair damaged cells and prevent the formation of wrinkle and spot.

The present invention also provides a method of using the multifunctional temperature self-regulating three-dimensional face mask. In some cases, the three-dimensional face mask not only can be used to warm/cool body, but also can be used to accelerate the absorption of skincare or healthcare products. In one embodiment, a skincare product or traditional nourishing mark is applied to the face prior to applying the present three-dimensional face mask; or, a skincare product is pre-coated on the surface of thermal-insulation layer in contact with the skin, the left side sheet and the right side sheet are adhered together, then the pre-coated three-dimensional face mask is applied to the face.

On the other hand, the present application provides a multifunctional face mask which can generate heat and absorb heat thereby to perform a hot compress and a cold compress, and can effectively prolong heating time and/or cooling time and reduce peak heating temperature and/or cooling temperature, and throughout which the energy is distributed evenly. The specific technical solution is as follows: a multifunctional temperature self-regulating face mask, from outside to inside comprising at least: a carrier layer loaded with functional material, and a thermal-insulation layer directly contacted with the skin; wherein the carrier layer loaded with functional material has a sealed cavity sealed with a flexible water-impermeable material, the sealed cavity is at least loaded with a refrigerating or heat-generating chemical material; wherein the thermal-insulation layer is made of a material having waterproof and thermal-insulation properties. The present multifunctional temperature self-regulating face mask is not limited to a structure of the above two layers, it may also comprise a package layer or adhesive layer etc. as needed.

Preferably, the thermal-insulation layer is made of metallized polyethylene terephthalate (MPET) film. Preferably, the flexible water-impermeable material is selected from plastics, synthetic rubbers, synthetic fibers, waterproof fabrics or waterproof papers.

In one embodiment, the sealed cavity of the face mask is loaded with a heat-generating chemical material which generates heat when the material changes from liquid to solid, and an actuating means which is used to trigger an exothermic state of the phase change material. According to the disclosure described above, the present face mask is capable of generating heat. Preferably, after the liquid-to-solid phase change process of the solid-liquid phase change material has been triggered, the multilayer face mask maintains a heating temperature at 40-55 °C, and the heating time lasts 5-30 minutes.

Preferably, the solid-liquid phase change material is an oversaturated aqueous solution of sodium acetate, and the actuating means is a flexible metal disc which takes a shape of an arc and has a plurality of corrugations on its surface, so as to be easily bent, and the flexible metal disc is located in the oversaturated aqueous solution of sodium acetate. Preferably, the oversaturated aqueous solution of sodium acetate is added with nucleating agent. Preferably, the nucleating agent is selected from one or more of the group consisting of sodium borate, sodium sulfate, sodium silicate, sodium pyrophosphate, sodium phosphate dibasic dodecahydrate.

Preferably, at least one side of the carrier layer is provided with a fluid reservoir. The fluid reservoir and the carrier layer are manufactured in one piece, and they are interconnected by the existing one-way valve. The fluid reservoir is loaded with fluid, when the fluid reservoirs is pressed by an external force, the fluid loaded in the fluid reservoir is injected into the carrier layer through the one-way valve to adjust the temperature of the carrier layer. According to the disclosure described above, the present face mask has both refrigerating and heat-generating functions.

In a specific embodiment, the carrier layer is loaded with a solid refrigerating chemical material, which absorbs heat when the material changes from solid to liquid. At least one side of the carrier layer is provided with a fluid reservoir. The fluid reservoir and the carrier layer are manufactured in one piece, and they are interconnected by the existing one-way valve. The fluid reservoir is loaded with fluid, when the fluid reservoir is pressed by an external force, the fluid loaded in the fluid reservoir is injected into the carrier layer through the one-way valve to dissolve the solid refrigerating chemical material, which would absorb energy. According to the disclosure described above, the present face mask has refrigerating function.

Preferably, after the solid-to-liquid phase change process of the solid refrigerating chemical material has been triggered, the face mask maintains a cooling temperature at -3-20°C, and the cooling time lasts 5-30 minutes. Preferably, the solid refrigerating chemical material which absorbs heat when the material changes from solid to liquid is urea, ammonium chloride, ammonium nitrate or mixture thereof, and urea is more preferable. The fluid loaded in the fluid reservoir is clean water. The weight ratio of the solid refrigerating chemical material to the clean water is 1:1.

Preferably, a suitable amount of thickener is added in the fluid loaded in the fluid reservoir of the face mask. Preferably, the carrier layer and/or the fluid reservoir is further loaded with a pigment.

Preferably, the face mask is 2-15mm thick. Preferably, in order to adhere the present face mask to the face, there is provided a layer of water-soluble hydrogel on the surface of the thermal-insulation layer that is in contact with the skin.

In one embodiment, the face mask has notches corresponding to the user's nose and mouth, respectively, so as to allow the user to breath during use of the face mask, and there is no notch provided on the portion corresponding to the user's eyes, so that the face mask can clean and rejuvenate most areas of the face expect the mouth and nose. In addition, the heat-generating layer and thermal-insulation layer of the face mask can warm and nourish the user's eyes, and prevent dry eye syndrome.

In another embodiment, besides the notches corresponding respectively to the user's nose and mouth, the face mask also has notches corresponding to the user's eyes, so that the user's sight and movement are not affected during use. Further, there is a fixing strap provided on either side of the face mask, and there is a velcro provided at the end of the fixing strap.

The present multifunctional face mask not only can be used for a hot compress or a cold compress, but also can be used for alternate hot and hold compress, so as to meet different needs of users, and it also has the technical advantages as mentioned above.

The present invention also provides a method of using the multifunctional temperature self-regulating face mask. In some cases, the face mask not only can be used to warm/cool body, but also can be used to accelerate the absorption of skincare or healthcare products. In one embodiment, a skincare product or traditional nourishing mark is applied to the face prior to applying the present face mask; or, a skincare product is pre-coated on the surface of thermal-insulation layer in contact with the skin, then the pre-coated face mask is applied to the face.

On the other hand, the present application provides a multifunctional eye mask which can generate heat and absorb heat thereby to perform a hot compress and a cold compress, and can effectively prolong heating time and/or cooling time and reduce peak heating temperature and/or cooling temperature, and throughout which the energy is distributed evenly. The specific technical solution is as follows: a multifunctional temperature self-regulating eye mask , from outside to inside comprising at least: a carrier layer loaded with functional material, and a thermal-insulation layer directly contacted with the skin; wherein the carrier layer loaded with functional material has a sealed cavity sealed with a flexible water-impermeable material, the sealed cavity is at least loaded with a refrigerating or heat-generating chemical material; wherein the thermal-insulation layer is made of a material having waterproof and thermal-insulation properties. The present multifunctional temperature self-regulating eye mask is not limited to a structure of the above two layers, it may also comprise a package layer or adhesive layer etc. as needed.

Preferably, the thermal-insulation layer is made of metallized polyethylene terephthalate (MPET) film. Preferably, the flexible water-impermeable material is selected from plastics, synthetic rubbers, synthetic fibers, waterproof fabrics or waterproof papers.

In one embodiment, the sealed cavity of the eye mask is loaded with a heat-generating chemical material which generates heat when the material changes from liquid to solid, and an actuating means which is used to trigger an exothermic state of the phase change material. According to the disclosure described above, the present eye mask is capable of generating heat. Preferably, after the liquid-to-solid phase change process of the solid-liquid phase change material has been triggered, the multilayer eye mask maintains a heating temperature at 40-55°C, and the heating time lasts 5-30 minutes.

Preferably, the solid-liquid phase change material is an oversaturated aqueous solution of sodium acetate, and the actuating means is a flexible metal disc which takes a shape of an arc and has a plurality of corrugations on its surface, so as to be easily bent, and the flexible metal disc is located in the oversaturated aqueous solution of sodium acetate. Preferably, the oversaturated aqueous solution of sodium acetate is added with nucleating agent. Preferably, the nucleating agent is selected from one or more of the group consisting of sodium borate, sodium sulfate, sodium silicate, sodium pyrophosphate, sodium phosphate dibasic dodecahydrate.

Preferably, at least one side of the carrier layer is provided with a fluid reservoir. The fluid reservoir and the carrier layer are manufactured in one piece, and they are interconnected by the existing one-way valve. The fluid reservoir is loaded with fluid, when the fluid reservoirs is pressed by an external force, the fluid loaded in the fluid reservoir is injected into the carrier layer through the one-way valve to adjust the temperature of the carrier layer. According to the disclosure described above, the present eye mask has both the refrigerating and heat-generating functions.

In a specific embodiment, the carrier layer is loaded with solid refrigerating chemical material, which absorbs heat when the material changes from solid to liquid. At least one side of the carrier layer is provided with a fluid reservoir. The fluid reservoir and the carrier layer are manufactured in one piece, and they are interconnected by the existing one-way valve. The fluid reservoir is loaded with fluid, when the fluid reservoir is pressed by an external force, the fluid loaded in the fluid reservoir is injected into the carrier layer through the one-way valve to dissolve the solid refrigerating chemical material, which would absorb energy. According to the disclosure described above, the present eye mask has refrigerating function.

Preferably, after the solid-to-liquid phase change process of the solid refrigerating chemical material has been triggered, the eye mask maintains a cooling temperature at -3-20°C, and the cooling time lasts 10-30 minutes.

Preferably, the solid refrigerating chemical material which absorbs heat when the material changes from solid to liquid is urea, ammonium chloride, ammonium nitrate or mixture thereof, and urea is more preferable. The fluid loaded in the fluid reservoir is clean water. The weight ratio of the solid refrigerating chemical material to the clean water is 1:1.

Preferably, a suitable amount of thickener is added in the fluid loaded in the fluid reservoir of the eye mask. Preferably, the carrier layer and/or the fluid reservoir is further loaded with a pigment.

Preferably, the eye mask is 2-15mm thick. Preferably, in order to adhere the present eye mask to the eyes, there is provided a layer of water-soluble hydrogel on the surface of the thermal-insulation layer that is in contact with the skin.

In one embodiment, the eye mask comprises two hanging parts which have ears slits and can be hung on the user's ears. In use, the eye mask can be reliably attached to the user's eyes by the hanging parts, so the eye mask would not easily move or fall off from the eyes.

Preferably, a slit is vertically cut along a lower half of a central vertical axis of the eye mask. Due to this, after applied to the eyes, a left side piece and a right side piece of the eye mask can naturally splay outward on the portion of nose bridge, so as to fit the user's eyes in shape, that is, the left side piece and the right side piece of the eye mask are symmetrically arranged along the nose bridge and naturally splay outward to the two sides of the slit so as to be reliably adhered to the eyes.

The present multifunctional eye mask not only can be used for a hot compress or a cold compress, but also can be used for alternate hot and hold compress, so as to meet different needs of users, and it also has the technical advantages as mentioned above. In addition, an alternate hot and hold compress can relieve eye irritation and dry eye syndrome.

The present invention also provides a method of using the multifunctional temperature self-regulating eye mask. In some cases, the eye mask not only can be used to warm/cool body, but also can be used to accelerate the absorption of skincare or healthcare products. In one embodiment, a skincare product or traditional nourishing mark is applied to the face prior to applying the present eye mask; or, a skincare product is pre-coated on the surface of thermal-insulation layer in contact with the skin, then the pre-coated eye mask is applied to the eyes.

### BRIEF DESCRIPTION OF THE DRAWINGS

Figure 1 is a schematic view of a multifunctional temperature self-regulating multilayer film according to Example 1 of the present invention.
Figure 2 is a schematic view showing the multifunctional temperature self-regulating multilayer film of Figure 1, with the film being in use.
Figure 3 is a schematic view of a multifunctional temperature self-regulating multilayer film according to Example 2 of the present invention.
Figure 4 is a schematic view of a multifunctional temperature self-regulating multilayer film according to Example 3 of the present invention.
Figure 5 is a schematic view of a multifunctional temperature self-regulating multilayer film according to Example 4 of the present invention.
Figure 6 is a schematic view of a multifunctional temperature self-regulating multilayer film according to Example 5 of the present invention.
Figure 7 is a schematic view of a face mask made from the multifunctional temperature self-regulating multilayer film according to Example 6 of the present invention.
Figure 8 is a schematic showing the structure of the multifunctional temperature self-regulating three-dimensional face mask according to Example 7 of the present invention.
Figure 9 is a schematic view showing a three-dimensional face mask consisting of the right side sheet and left side sheet of Figure 8 when the right side sheet and left side sheet are adhered together.
Figure 10 is a schematic view showing a concave-convex shape of the three-dimensional face mask of Figure 8 when the three-dimensional face mask is attached to the user's face.
Figure 11 shows a sectional view of a face portion of the three-dimensional face mask of Figure 8, with the face portion having a structure of two layers.
Figure 12 is a schematic view showing the structure of a multifunctional temperature self-regulating three-dimensional face mask according to Example 8 of the present invention.
Figure 13 is a schematic view showing a three-dimensional face mask consisting of the right side sheet and left side sheet of Figure 12 when the right side sheet and left side sheet are adhered together.
Figure 14 is a schematic view showing a concave-convex shape of the three-dimensional face mask of Figure 12 when the three-dimensional face mask is attached to the user's face.
Figure 15 is a schematic view showing the structure of a multifunctional temperature self-regulating three-dimensional face mask according to Example 10 of the present invention.
Figure 16 is a schematic view showing a three-dimensional face mask consisting of the right side sheet and left side sheet of Figure 17 when the right side sheet and left side sheet are adhered together.
Figure 17 is a schematic view showing the structure of a multifunctional temperature self-regulating three-dimensional face mask according to Example 11 of the present invention.
Figure 18 is a schematic view showing a three-dimensional face mask consisting of the right side sheet and left side sheet of Figure 17 when the right side sheet and left side sheet are adhered together.
Figure 19 is a schematic view of a multifunctional temperature self-regulating face mask according to Example 12 of the present invention.
Figure 20 shows a sectional view of a face portion of the face mask of Figure 19, with the face portion having a structure of two layers.
Figure 21 is a schematic view of a multifunctional temperature self-regulating face mask according to Example 13 of the present invention.
Figure 22 is a schematic view of a multifunctional temperature self-regulating face mask according to Example 15 of the present invention.
Figure 23 is a schematic view of a multifunctional temperature self-regulating face mask according to Example 16 of the present invention.
Figure 24 is a schematic view of a multifunctional temperature self-regulating eye mask according to Example 12 of the present invention.
Figure 25 shows a sectional view of an eye portion of the eye mask of Figure 24, with the eye portion having a structure of two layers.
Figure 26 is a schematic view of a multifunctional temperature self-regulating eye mask according to Example 18 of the present invention.
Figure 27 is a schematic view of a multifunctional temperature self-regulating eye mask according to Example 20 of the present invention.
Figure 28 is a schematic view of a multifunctional temperature self-regulating eye mask according to Example 21 of the present invention.

### DETAILED DESCRIPTION OF ILLUSTRATED EMBODIMENTS

The present invention will be described in detail in connection with the following preferred embodiments, and it will be appreciated that such embodiments are merely exemplary, the invention is not to be limited to the disclosed embodiments, but on the contrary, is intended to cover various modifications and equivalent arrangements included within the spirit and scope thereof.

### Example 1

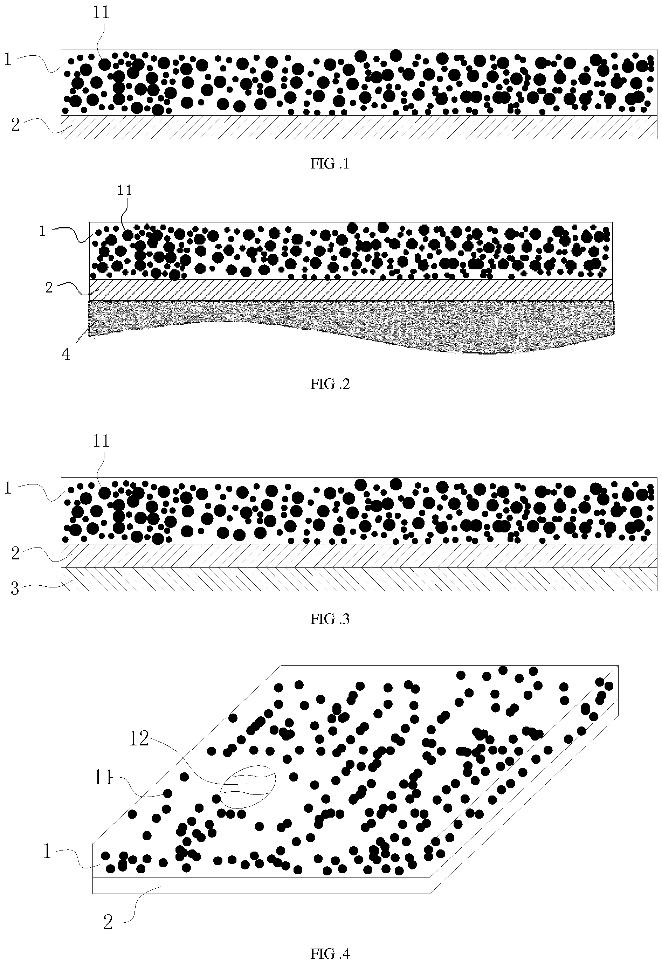

As shown in Figure 1, the multifunctional temperature self-regulating multilayer film comprises a structure of two layers: a carrier layer 1 loaded with functional material and a thermal-insulation layer 2; wherein the carrier layer 1 is loaded with refrigerating or heat-generating chemical material 11. Figure 2 is a schematic view showing the state during use of the multifunctional temperature self-regulating multilayer film of Figure 1, the thermal-insulation layer 2 is in contact with the skin 4.

In this example, the carrier layer 1 loaded with functional material and the thermal-insulation layer 2 are adhered together by, for example, the adhesive or thermal bonding, so they are applied to the skin as a whole. In some cases, the thermal-insulation layer 2 is not adhered together with the carrier layer 1 loaded with functional material. That is, in use, the thermal-insulation layer is first applied to the skin, then the carrier layer 1 loaded with functional material is applied, and the similar effect can also be obtained.

In this example, the carrier layer 1 loaded with functional material is made of transparent PVC material. In practice, just be sure that the carrier layer 1 loaded with functional material is made of transparent and flexible water-impermeable material, the existing water-impermeable fabrics, papers and synthetic fibers etc. can be suitable. The volume of the carrier layer 1 loaded with functional material varies depending upon the refrigerating or heat-generating chemical material and the amount thereof, and the desired heating/ cooling temperature. The shape of the present film depends upon the body part to be applied to. The thermal-insulation layer 2 is formed by a PET aluminum film made of PET and silvery aluminum. Aluminum has a lower melting point, so it can be easily changed from solid to gas by heating under vacuum. Compared with other film, PET film has better heat resistance and compatibility with aluminum. PET aluminum film has a reflective silvery surface, which can reflect heat back. The heat reflectivity of a PET aluminum film with a thickness of 400A°is almost 90%, so it has excellent heat-insulation effect. In addition, PET aluminum film also has good water and gas barrier property.

### Example 2

Figure 3 is a schematic view of a multifunctional temperature self-regulating multilayer film according to Example 2 of the present invention. Compared with Example 1, there is also provided a water-soluble hydrogel layer 3 on the surface of the thermal-insulation layer 2 that is in contact with the skin, so as to adhere the multilayer film to the user's body. In practice, the water-soluble hydrogel layer 3 may comprise an active ingredient of a topically-applied healthcare product, a topically-applied medicine or a skincare product.

### Example 3

Figure 4 is a schematic view of a multifunctional temperature self-regulating multilayer film according to Example 3 of the present invention. In this example, the functional material 11 loaded in the carrier layer 1 is a sodium acetate solution at a concentration of 80-90%, and a stainless steel disc 12 which is concave in shape is also loaded in the carrier layer 1. The carrier layer 1 loaded with functional material is made of transparent OPP film, so the stainless steel disc 12 can be seen by the user. When a hot compress is needed, press the stainless steel disc 12 with the hand to cause sodium acetate to form crystals and release heat. In this example, the multilayer film can be reused when immersing it into hot water at about 80°C for 2-3 minutes to dissolve the crystals. In order to increases the using life of the film, the nucleating agent, 10 wt. % sodium sulfate, is added in the oversaturated aqueous solution of sodium acetate. In other examples, the nucleating agent may be 10 wt. % sodium silicate, sodium pyrophosphate, sodium phosphate dibasic dodecahydrate or mixture thereof. The present film can be reused for hundreds of times. In addition, a little red pigment is also added in the oversaturated aqueous solution of sodium acetate to improve the esthetic effect of the film.

### Example 4

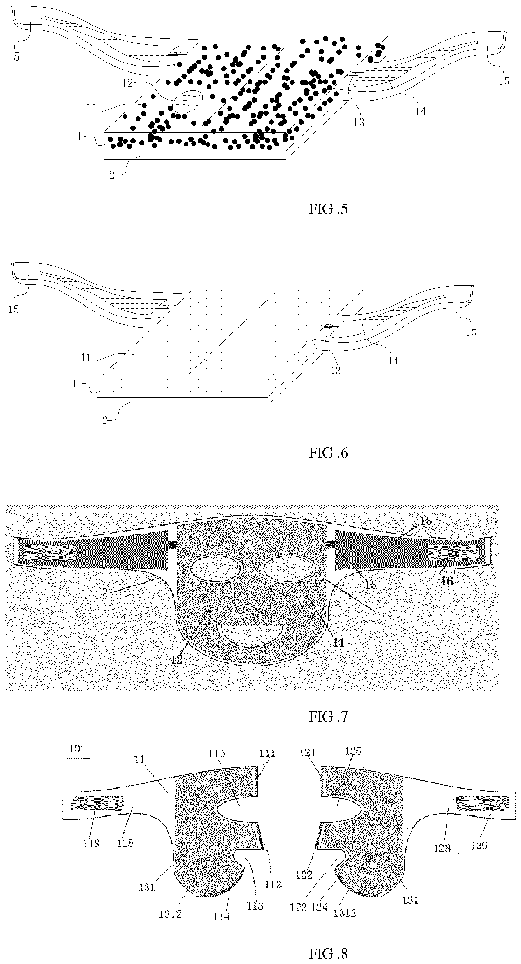

Figure 5 is a schematic view of a multifunctional temperature self-regulating multilayer film according to Example 4 of the present invention. Compared with Example 3, the carrier layer 1 loaded with functional material in Example 4 is provided with two fluid reservoirs 15 on its two sides. The fluid reservoir 15 is loaded with clean water 14. The fluid reservoir 15 and the carrier layer 1 are manufactured in one piece, and they are interconnected by the existing one-way valve 13. When a user wants to adjust the temperature of the carrier layer 1 loaded with functional material or wants a cold compress following a hot compress, he only needs to press the fluid reservoirs 15 with hand to make the clean water 14 loaded in the fluid reservoir 15 flow to the carrier layer 1 and mix with the sodium acetate crystals, then the cooling effect would be achieved due to endothermic reaction. In use, the addition of clean water 14 decreases the concentration of the oversaturated aqueous solution of sodium acetate, so the phase balance of the oversaturated aqueous solution of sodium acetate loaded in the carrier layer 1 is broken, when the film is used again later, the heating temperature is a little lower than before.

### Example 5

Figure 6 is a schematic view of a multifunctional temperature self-regulating multilayer film according to Example 5 of the present invention. In this example, the functional material 11 loaded in the carrier layer 1 is urea (120g). The fluid reservoir 15 is loaded with clean water 14 (120g), and the fluid reservoir 15 and the carrier layer 1 are manufactured in one piece, and they are interconnected by the existing one-way valve 13. When a user wants a cold compress, he only needs to press the fluid reservoirs 15 with hand to make the clean water 14 loaded in the fluid reservoir 15 flow to the carrier layer 1 and mix with urea, then the cooling effect would be achieved due to endothermic reaction.

### Example 6

Figure 7 is a schematic view of a face mask made from the multifunctional temperature self-regulating multilayer film according to Example 6 of the present invention. In this example, the functional material 11 loaded in the carrier layer 1 is a sodium acetate solution at a concentration of 80-90%, and the fluid reservoir 15 is made into a long and narrow strip shape. Either of the fluid reservoirs 15 is provided with a velcro 16 which is used to connect the two fluid reservoirs 15 together, so as to attach the face mask to the user's head. In other example, the velcro 16 may be replaced with a lacing or fastener extending from the fluid reservoir 15. The clean water injected into the carrier layer 1 may accumulate at the bottom of the film due to gravity, which may cause an uneven temperature distribution. In order to avoid this, a little thickener (such as pectin) is added in the clean water 14. In use, the user should press the stainless steel disc 12 to cause the face mask to release heat and apply a skincare product to the face, then apply the face mask to the face; and after the face mask has cooled to room temperature, he can press the fluid reservoirs 15 with hand to make the clean water 14 loaded in the fluid reservoir 15 flow to the carrier layer 1 and mix with the sodium acetate crystals, the process would absorb heat and refrigerate the face mask; the refrigerated face mask can be applied to the face again to cool the face skin. By the above processes, a cold compress following a hot compress can be available, which can improve the absorption of the skincare products and shrink the skin pores.

### Comparative Example 1

The face mask in Comparative Example 1 is similar to that in Example 6 except that no thermal-insulation layer 2 is provided, and it is used in the same way as described in Example 6.

The results show that the duration of the heat generation (in a heat range above 37°C) for the face mask in Example 6 is about 35 minutes, while it is only about 20 minutes for the face mask in Comparative Example 1; and the duration of the refrigeration for the face mask in Example 6 is about 30 minutes, while it is only about 20 minutes for the face mask in Comparative Example 1. In addition, it has been demonstrated in experiments that due to the thermal-insulation layer according to the invention the heat generation/refrigeration in Example 6 is significantly more homogeneous than that in Comparative Example 1, that is, the face mask in Example 6 overcomes the problem of the face mask in Comparative Example 1 which is either too hot or too cold for most of the time it is being used. Compared with Comparative Example 1, the face mask in Example 6 improves the absorption effect of the skincare and the moisture content of the skin.

### Example 7

The example mainly relates to a three-dimensional face mask which has a facial concave-convex shape formed by joining two sheets together. Compared with the traditional face mask made from a single sheet of material, the three-dimensional face mask can fit the face better, so as to uniformly clean and nourish the face skin.

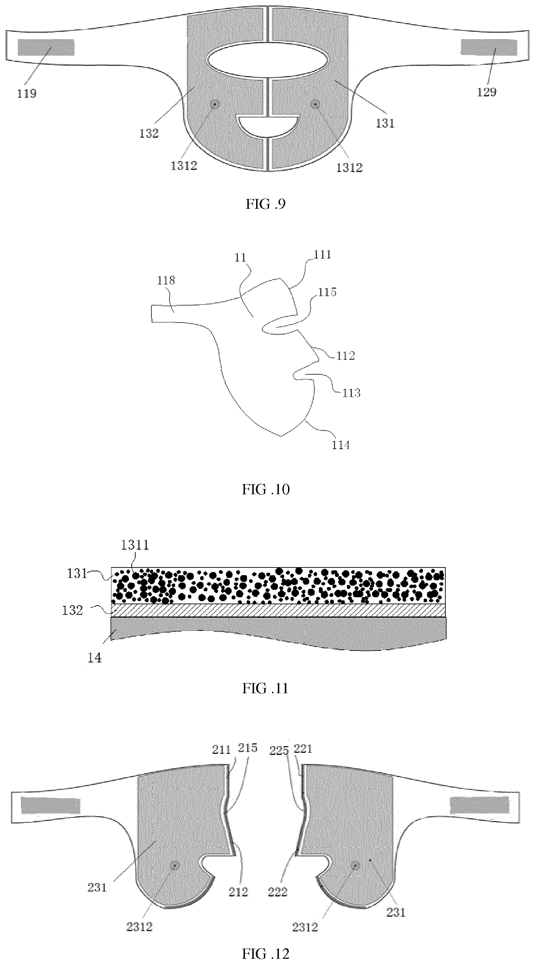

As shown in Figure 8, the three-dimensional face mask 10 comprises a left side sheet 11 and a right side sheet 12 respectively matching the left area of the face and the right area of the face. Either of the front edge portions 111, 121 of the left side sheet 11 and the right side sheet 12 is provided with an adhesive material, and the left side sheet 11 and the right side sheet 12 are thereby adhered together by adhesive material along the median line of the face. Figure 9 is a schematic view showing a face mask consisting of the right side sheet and left side sheet of Figure 8 when the right side sheet and left side sheet are adhered together.

In this example, the left side sheet 11 and the right side sheet 12 are symmetrical along the median line of the face. Of course, the present application is not limited to this, the left side sheet 11 and the right side sheet 12 may also be unsymmetrical in other examples. However, from the perspective of somatology, it is easier to fit the face when the two sheets are symmetrical. Since the two sheets are adhered together to form a facial concave-convex shape, it is possible to keep this three-dimensional face mask completely adhering to every facial curve, so as to overcome the deficiencies of the traditional face mask, which is built from a single mask sheet and is liable to wrinkle and slack, thereby the traditional one-piece face mask cannot be uniformly and reliably applied to the face and affects the cosmetic and cleaning effect.

As shown in Figure 8, either of the front edge portion 111 of the left side sheet 11 and the front edge portion 121 of the right side sheet 12 is provided with a protrusion 112,122 projecting outwards from above down and matching the shape of the left area or right area of the external nose. A notch 113,123 corresponding to the mouth is respectively provided below the protrusion 112, 122. After the two sheets are joined together, the notch 113, 123 are joined and form a mouth portion matching the mouth. There is no adhesive material provided on the notch 113, 123 of the front edge portion 111,121. The portion of the front edge portion below the notch 113,123 respectively has an arc shape 114,124 with a certain radian and extends downwards to the bottom the face mask. Since either of the front edge portion 111 of the left side sheet 11 and front edge portion 121 of the right side sheet 12 has a protrusion projecting outwards from above down and matching the shape of the left area or right area of the external nose, after the left side sheet 11 and the right side sheet 12 are joined together, the three-dimensional face mask would naturally have a nose-like protrusion from above down on the user's nose, so as to reliably fit the nose. Since the portion of the front edge portion 111, 121 below the notch 113, 123 respectively has an arc shape with a certain radian and extends downwards to the bottom the face mask, after the left side sheet 11 and the right side sheet 12 are joined together, the lower half of the face mask has a curved surface which can reliably fit the jaw, so as to improve the adherence to the face.

Figure 10 shows the concave-convex shape of the three dimensional face mask which has been applied to the user's face. A nose-like protrusion is formed after the protrusion 112, 122 are joined together, and an elliptical notch corresponding to the mouth is formed after the notch 113, 123 are joined together, and a curved surface is formed after the arc shape 114,124 are joined together, so as to fit the lower face and jaw. It can be seen that compared with the traditional sheet-mask, the present three-dimensional face mask fits the user's face which has a concave-convex shape better, so that it can be uniformly applied to the entire face and uniformly clean and nourish the face skin.

Preferably, a notch 115,125 which is concaved inwards is provided on the eye-and-glabella portion of either of the front edge portion 111 , 121 of the left side sheet 11 and the right side sheet 12 which faces the user's eyes and glabella, just above the protrusion 112, 122. The notch 114, 124 can be almost semielliptical or semicircular, as long as the eyes are uncovered but the skin around the eyes is almost covered. On either of the front edge portions 111,121 of the left side sheet 11 and the right side sheet 12, neither the notch 113, 123 corresponding to the mouth nor the notch 115,125 corresponding to the eye-and-glabella is provided with adhesive material. That is, the left side sheet and the right side sheet are provided with an adhesive material on either of the front edge portions, except the notch 113, 123 corresponding to the mouth and the notch 115,125 corresponding to the eye-and-glabella, and the left side sheet and the right side sheet are thereby adhered together by the adhesive material.

Preferably, as shown in Figure 8, the rear edge portions of the left side sheet 11 and the right side sheet 12 are respectively provided with a fixing strap 118, 128, and preferably, the fixing strap 118, 128 are respectively provided with a velcro 119, 129. After applied to the face, the three-dimensional face mask can be attached to the user's head by the fixing strap and tighten by the velcro. The velcro, also known as hook-and-loop or hook-and-pile, is a common fastener used on the clothes, which is consisted of two components, typically, the first component features tiny hooks; the second features even smaller and "hairier" loops.

In this example, as shown in Figure 11, either of the face portions 116, 126 of the left side sheet 11 and the right side sheet 12 of the three-dimensional face mask 10 comprises a structure of two layers: a carrier layer 131 loaded with functional material and a thermal-insulation layer 132; wherein the carrier layer 131 is loaded with refrigerating or heat-generating chemical material 1311. As shown in Figure 11, the thermal-insulation layer 132 is in contact with the skin 14.

In this example, the carrier layer 131 loaded with functional material and the thermal-insulation layer 132 are adhered together by, for example, the adhesive or thermal bonding, so they are applied to the skin as a whole. In some cases, the thermal-insulation layer 132 is not adhered together with the carrier layer 131 loaded with functional material. That is, in use, the thermal-insulation layer is first applied to the skin, then the carrier layer 131 loaded with functional material is applied, and the similar effect can also be obtained.

In this example, the carrier layer 131 loaded with functional material is made of transparent PVC material. In this example, the functional material 1311 loaded in the carrier layer 131 is a sodium acetate solution at a concentration of 80-90%, and a stainless steel disc 1312 which is concave in shape is also loaded in the carrier layer 131. The carrier layer 131 loaded with functional material is made of transparent OPP film, so the stainless steel disc 1312 can be seen by the user. When a hot compress is needed, press the stainless steel disc 1312 with the hand to cause sodium acetate to form crystals and release heat.

In this example, the thermal-insulation layer 132 is formed by a PET aluminum film made of PET and silvery aluminum.

### Example 8

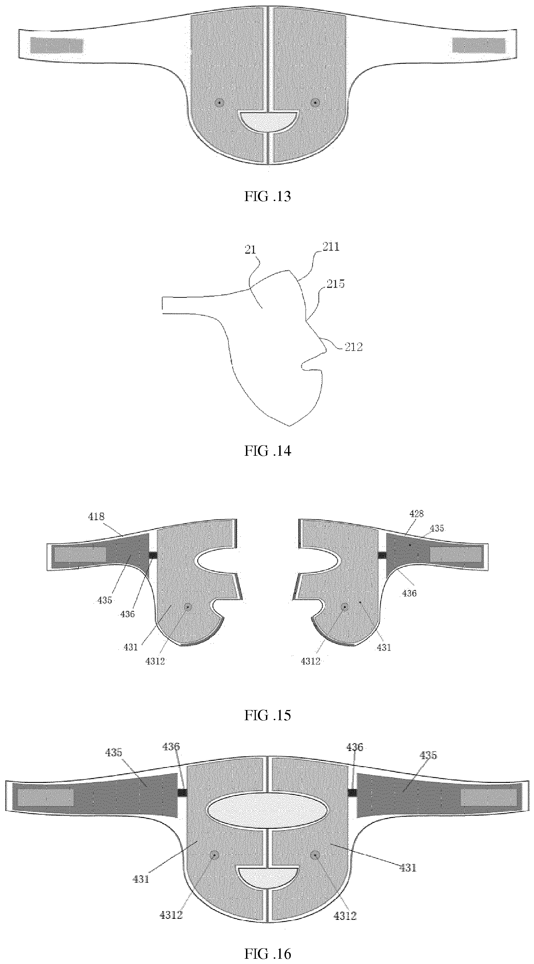

Figures 12-14 show the structure of a multifunctional temperature self-regulating three-dimensional face mask according to Example 8. The difference of Example 8 over example 7 is that the three-dimensional face mask in Example 8 is inclosed except that there is provided a notch corresponding to the user's mouth. That is to say, there is no notch provided on the eye and eyebrow portions of the front edge portion 211 of the left side sheet 21 and the front edge portion 221 of the right side sheet 22. In this example, either of the glabella portions of the front edge portions 211, 221 of the left side sheet 21 and the right side sheet 22 which faces a space between the user's eyebrows, just above the protrusion 212, 222, is formed as an arc 215, 225 which is slightly concaved inwards. The arc 215, 225 is provided with an adhesive material. Due to the arc 215, 225, after the left side sheet 21 and the right side sheet 22 are joined together, the three-dimensional face mask would have a slight concave on the arc 215, 225, such that the three-dimensional face mask reliably fits the concave on the user's glabella-and-nasion portion, just as shown in Figure 14. It should be note that drawing reference 231 represents the carrier layer loaded with functional material and drawing reference 2311 represents the actuating means.

The three-dimensional face mask in this example only has a notch on the portion corresponding to the mouth (including the portion below the nostril), other areas are inclosed, so it can clean and rejuvenate most areas of the face.

### Example 9

The difference of Example 9 over Example 8 is that there is provided a layer of water-soluble hydrogel on the surface of the thermal-insulation layer 132 that is in contact with the skin.

### Example 10

As shown in Figures 15-16, the difference of Example 10 over Example 8 is that the carrier layer 431 loaded with functional material is provided with two fluid reservoirs 435 on its two sides. In this example, the two fluid reservoirs 435 are respectively arranged on the left fixing strap 418 and the right fixing strap 428. The fluid reservoir 435 is loaded with clean water. The fluid reservoir 435 and the carrier layer 431 are manufactured in one piece, and they are interconnected by the existing one-way valve 436. When a user wants to adjust the temperature of the carrier layer 431 loaded with functional material or wants a cold compress following a hot compress, he only needs to press the fluid reservoirs 435 with hand to make the clean water loaded in the fluid reservoir 435 flow to the carrier layer 431 and mix with the sodium acetate crystals, then the cooling effect would be achieved due to endothermic reaction. In use, the addition of clean water decreases the concentration of the oversaturated aqueous solution of sodium acetate, so the phase balance of the oversaturated aqueous solution of sodium acetate loaded in the carrier layer 431 is broken, when the film is used again later, the heating temperature is a little lower than before. The clean water injected into the carrier layer may accumulate at the bottom of the face mask, which may cause an uneven temperature distribution. In order to avoid this, a little thickener (such as pectin) is added in the clean water.

In use, the user should press the stainless steel disc 4312 to cause the face mask to release heat and apply a skincare product to the face, then apply the face mask to the face; and after the face mask has cooled to room temperature, he can press the fluid reservoirs 435 with hand to make the clean water loaded in the fluid reservoir 435 flow to the carrier layer 4331 and mix with the sodium acetate crystals, the process would absorb heat and refrigerate the face mask; the refrigerated face mask can be applied to the face again to cool the face skin. By the above processes, a cold compress following a hot compress can be available.

### Example 11

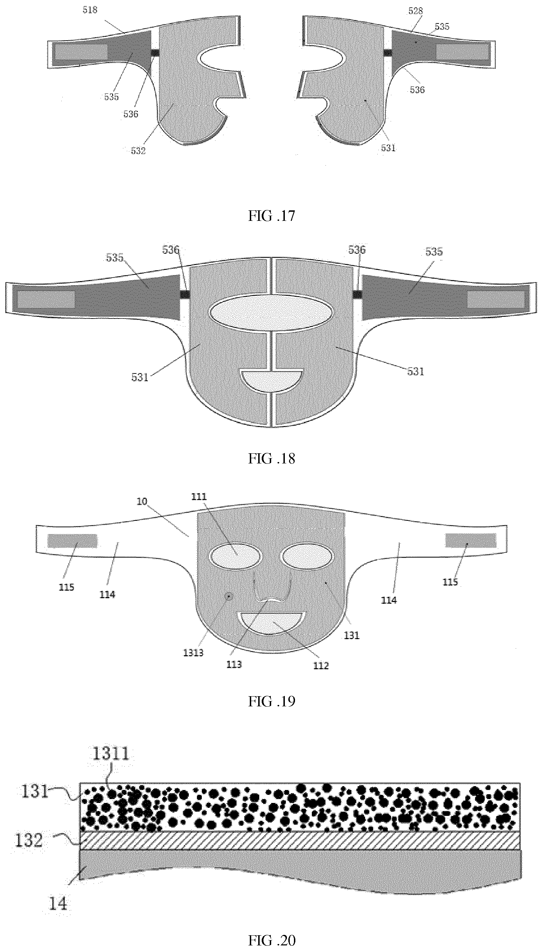

Figures 17-18 show a multifunctional temperature self-regulating three-dimensional face mask according to Example 11 of the present invention. In this example, the functional material loaded in the carrier layer 531 is urea (120g). The fluid reservoir 535 is loaded with clean water (120g), and the fluid reservoir 535 and the carrier layer 531 are manufactured in one piece, and they are interconnected by the existing one-way valve 536. Just as Example 10, the two fluid reservoirs 535 are respectively arranged on the left fixing strap 518 and the right fixing strap 528. When a user wants a cold compress, he only needs to press the fluid reservoirs 535 with hand to make the clean water loaded in the fluid reservoir 535 flow to the carrier layer 531 and mix with urea, then the cooling effect would be achieved due to endothermic reaction. The face mask in Example 11 is only used for a cold compress, so there is no actuating means provided in the three-dimensional face mask.

### Comparative Example 2

The face mask in Comparative Example 2 is similar to that in Example 10 except that no thermal-insulation layer is provided, and it is used in the same way as described in Example 10.

The results show that the duration of the heat generation (in a heat range above 37°C) for the face mask in Example 10 is about 35 minutes, while it is only about 20 minutes for the face mask in Comparative Example 2; and the duration of the refrigeration for the face mask in Example 10 is about 30 minutes, while it is only about 20 minutes for the face mask in Comparative Example 2. In addition, it has been demonstrated in experiments that due to the thermal-insulation layer according to the invention the heat generation/refrigeration in Example 10 is significantly more homogeneous than that in Comparative Example 2, that is, the face mask in Example 10 overcomes the problem of the face mask in Comparative Example 2 which is either too hot or too cold for most of the time it is being used. Compared with Comparative Example 2, the face mask in Example 10 improves the absorption effect of the skincare and the moisture content of the skin.

### Example 12

As shown in Figure 19, in Example 12, the face mask 10 has notches 111 corresponding to the user's eyes, and notch 112 corresponding to the mouth, and notch 113 corresponding to the nostril. The two sides of the face mask 10 are provided with a fixing strap 114, and the fixing strap 114 is provided with a velcro 115.

After applied to the face, the face mask 10 can be attached to the user's head by the fixing strap 114 and tighten by the velcro 115 .

In this example, as shown in Figure 19, the face portion of the face mask 10 comprises a structure of two layers: a carrier layer 131 loaded with functional material and a thermal-insulation layer 132; wherein the carrier layer 131 is loaded with refrigerating or heat-generating chemical material 1311. As shown in Figure 20, the thermal-insulation layer 132 is in contact with the skin 14.

In this example, the carrier layer 131 loaded with functional material and the thermal-insulation layer 132 are adhered together by, for example, the adhesive or thermal bonding, so they are applied to the skin as a whole. In some cases, the thermal-insulation layer 132 is not adhered together with the carrier layer 131 loaded with functional material. That is, in use, the thermal-insulation layer is first applied to the skin, then the carrier layer 131 loaded with functional material is applied, and the similar effect can also be obtained.

In this example, the carrier layer 131 loaded with functional material is made of transparent PVC material. In this example, the functional material 1311 loaded in the carrier layer 131 is a sodium acetate solution at a concentration of 80-90%, and a stainless steel disc 1312 which is concave in shape is also loaded in the carrier layer 131. The carrier layer 131 loaded with functional material is made of transparent OPP film, so the stainless steel disc 1312 can be seen by the user. When a hot compress is needed, press the stainless steel disc 1312 with the hand to cause sodium acetate to form crystals and release heat.

In this example, the thermal-insulation layer 132 is formed by a PET aluminum film made of PET and silvery aluminum.

### Example 13

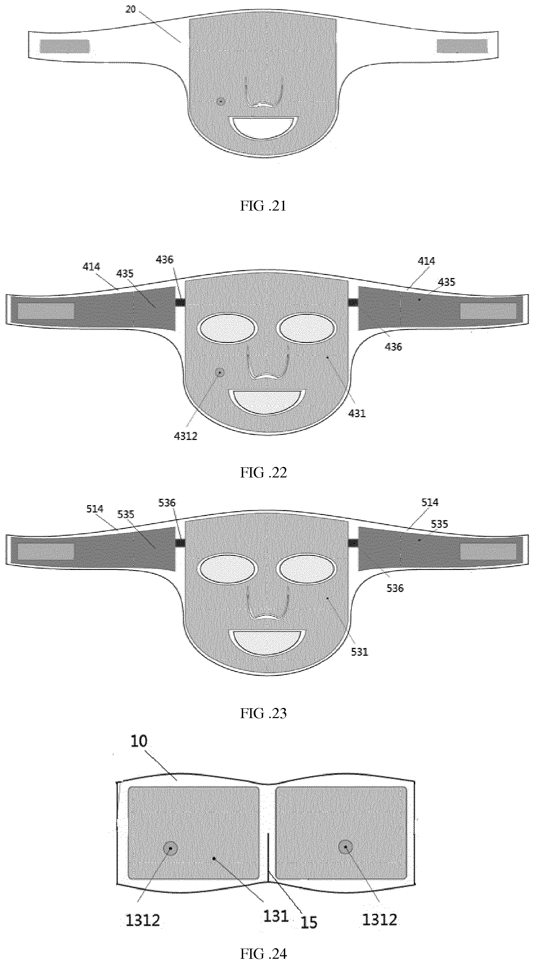

Figure 21 shows a multifunctional temperature self-regulating face mask according to Example 13 of the present invention. The difference of Example 13 over Example 12 is that there is no notch provided on the portions corresponding to the user's eyes. That is to say, the face mask is inclosed on eye portions. Since there is no notch on the portions corresponding to the user's eyes, so the face mask can clean and rejuvenate most areas of the face except the mouth and nostril.

### Example 14

The difference of Example 14 over Example 12 is that there is provided a layer of water-soluble hydrogel on the surface of the thermal-insulation layer 132 that is in contact with the skin, which is used for adhering the face mask to the skin.

### Example 15

As shown in Figure 22, the difference of Example 15 over Example 12 is that the carrier layer 431 loaded with functional material is provided with two fluid reservoirs 435 on its two sides. In this example, the two fluid reservoirs 435 are respectively arranged on the two fixing straps 414. The fluid reservoir 435 is loaded with clean water. The fluid reservoir 435 and the carrier layer 431 are manufactured in one piece, and they are interconnected by the existing one-way valve 436. When a user wants to adjust the temperature of the carrier layer 431 loaded with functional material or wants a cold compress following a hot compress, he only needs to press the fluid reservoirs 435 with hand to make the clean water loaded in the fluid reservoir 435 flow to the carrier layer 431 and mix with the sodium acetate crystals, then the cooling effect would be achieved due to endothermic reaction. In use, the addition of clean water decreases the concentration of the oversaturated aqueous solution of sodium acetate, so the phase balance of the oversaturated aqueous solution of sodium acetate loaded in the carrier layer 431 is broken, when the film is used again later, the heating temperature is a little lower than before. The clean water injected into the carrier layer may accumulate at the bottom of the face mask, which may cause an uneven temperature distribution. In order to avoid this, a little thickener (such as pectin) is added in the clean water.

In use, the user should press the stainless steel disc 4312 to cause the face mask to release heat and apply a skincare product to the face, then apply the face mask to the face; and after the face mask has cooled to room temperature, he can press the fluid reservoirs 435 with hand to make the clean water loaded in the fluid reservoir 435 flow to the carrier layer 4331 and mix with the sodium acetate crystals, the process would absorb heat and refrigerate the face mask; the refrigerated face mask can be applied to the face again to cool the face skin. By the above processes, a cold compress following a hot compress can be available.

### Example 16

Figure 23 shows a multifunctional temperature self-regulating face mask according to Example 16 of the present invention. In this example, the functional material loaded in the carrier layer 531 is urea (120g). The fluid reservoir 535 is loaded with clean water (120g), and the fluid reservoir 535 and the carrier layer 531 are manufactured in one piece, and they are interconnected by the existing one-way valve 536. Just as Example 15, the two fluid reservoirs 535 are respectively arranged on the fixing straps 514. When a user wants a cold compress, he only needs to press the fluid reservoirs 535 with hand to make the clean water loaded in the fluid reservoir 535 flow to the carrier layer 531 and mix with urea, then the cooling effect would be achieved due to endothermic reaction. The face mask in Example 11 is only used for a cold compress, so there is no actuating means provided in the face mask.

### Comparative Example 3

The face mask in Comparative Example 3 is similar to that in Example 15 except that no thermal-insulation layer is provided, and it is used in the same way as described in Example 15.

The results show that the duration of the heat generation (in a heat range above 37°C) for the face mask in Example 15 is about 35 minutes, while it is only about 20 minutes for the face mask in Comparative Example 3; and the duration of the refrigeration for the face mask in Example 15 is about 30 minutes, while it is only about 20 minutes for the face mask in Comparative Example 3. In addition, it has been demonstrated in experiments that due to the thermal-insulation layer according to the invention the heat generation/refrigeration in Example 15 is significantly more homogeneous than that in Comparative Example 3, that is, the face mask in Example 15 overcomes the problem of the face mask in Comparative Example 3 which is either too hot or too cold for most of the time it is being used. Compared with Comparative Example 3, the face mask in Example 15 improves the absorption effect of the skincare and the moisture content of the skin.

### Example 17

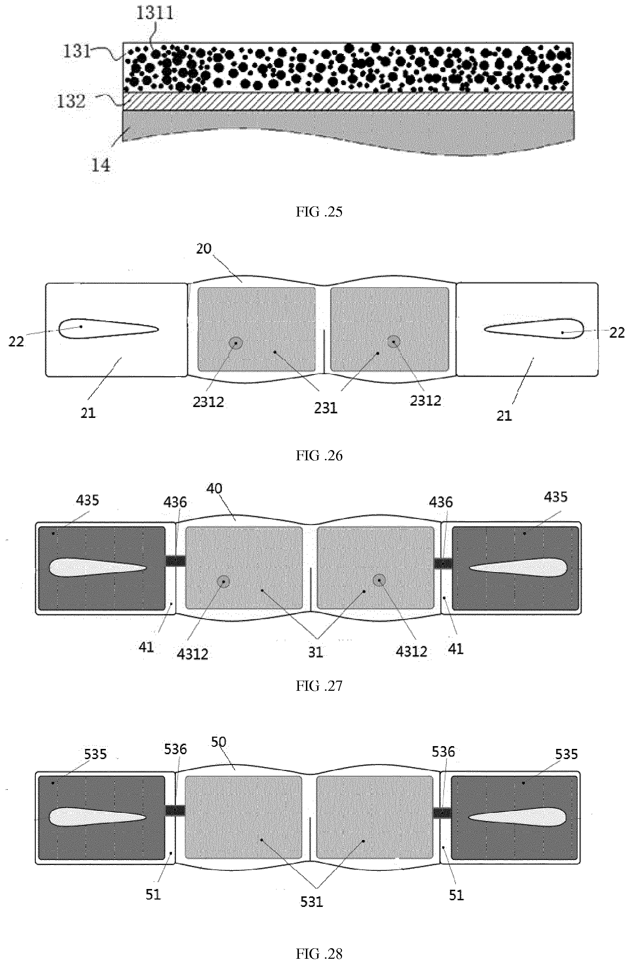

As shown in Figures 24-25, in this example, the eye portion of the eye mask 10 comprises a structure of two layers: a carrier layer 131 loaded with functional material and a thermal-insulation layer 132; wherein the carrier layer 131 is loaded with refrigerating or heat-generating chemical material 1311. As shown in Figure 25, the thermal-insulation layer 132 is in contact with the skin 14.

In this example, the carrier layer 131 loaded with functional material and the thermal-insulation layer 132 are adhered together by, for example, the adhesive or thermal bonding, so they are applied to the skin as a whole. In some cases, the thermal-insulation layer 132 is not adhered together with the carrier layer 131 loaded with functional material.

In this example, the carrier layer 131 loaded with functional material is made of transparent PVC material. In this example, the functional material 1311 loaded in the carrier layer 131 is a sodium acetate solution at a concentration of 80-90%, and a stainless steel disc 1312 which is concave in shape is also loaded in the carrier layer 131. The carrier layer 131 loaded with functional material is made of transparent OPP film, so the stainless steel disc 1312 can be seen by the user. When a hot compress is needed, press the stainless steel disc 1312 with the hand to cause sodium acetate to form crystals and release heat. In this example, the eye mask can be reused when immersing it into hot water at about 80°C for 2-3 minutes to dissolve the crystals. In order to increases the using life of the eye mask, the nucleating agent, 10 wt. % sodium silicate, is added in the oversaturated aqueous solution of sodium acetate.

In this example, the thermal-insulation layer 132 is formed by a PET aluminum film made of PET and silvery aluminum.

In this example, the heat given off from the carrier layer loaded with functional material can relax the muscle around the eyes, enlarge blood vessel and accelerate blood circulation, so as to relieve swelling, dry eye syndrome and eye irritation. In addition, a thermal-insulation layer formed of a material having waterproof and thermal-insulation properties is provided between the carrier layer and the skin. When used, the thermal-insulation layer ensures the heat given off from the carrier layer loaded with functional material is evenly and stably transferred to the skin, and the heat will not spread out quickly from the skin, so as to avoid scalding caused by too high initial temperature and maintain stable and suitable holding temperature. Further, the thermal-insulation layer prevents the water or oil in the skincare product, external medicine or healthcare product or sweat from permeating into the functional material, so as to avoid uncontrollable peak heating temperature and cross-contamination between the chemical reaction products of the functional material and the water or oil in the skincare product, or healthcare product or the sweat, which may cause skin infection. When applied to the face, the heat given off by the carrier layer loaded with functional material and maintained by the thermal-insulation layer makes the skin comfortable, improves blood circulation and enlarges facial pores, so as to clean skin dirt and sebaceous secretion. When used in combination with a skincare product, the absorption of the effective ingredients in said traditional eye mask or skincare product is improved, so the amount of skincare product used is reduced and skincare effect is enhanced. In addition, the use is convenient, environmentally friendly and low-cost.

In this example, a slit 15 is vertically cut along a lower half of a central vertical axis of the eye mask. Due to this, after applied to the eyes, a left side piece and a right side piece of the eye mask can naturally splay outward on the portion of nose bridge, so as to fit the user's eyes in shape, that is, the left side piece and the right side piece of the eye mask are symmetrically arranged along the nose bridge and naturally splay outward to the two sides of the slit so as to be reliably adhered to the eyes.

### Example 18

Figure 26 shows a multifunctional temperature self-regulating eye mask according to Example 18 of the present invention. The difference of Example 18 over Example 17 is that the eye mask 20 is also provided with two hanging parts 21, and the hanging parts 21 have ears slits 22 which can hung on the user's ears. In use, the eye mask can be reliably attached to the user's eyes by the hanging parts 21, so the eye mask would not easily move or fall off from the eyes.

### Example 19

In this example, there is provided a layer of water-soluble hydrogel on the surface of the thermal-insulation layer 132 of the eye mask that is in contact with the skin, so as to adhere the multilayer film to the user's body. In practice, the water-soluble hydrogel layer may comprise an active ingredient of a topically-applied healthcare product, a topically-applied medicine or a skincare product. Since the present multilayer film comprises an additional thermal-insulation layer formed of a material having waterproof and thermal-insulation properties, the water in the hydrogel can't permeate into the carrier layer, so as to avoid affecting peak heating/cooling temperature and heating/cooling effect.

### Example 20

As shown in Figure 27, the difference of Example 20 over Example 17 is that the carrier layer 431 loaded with functional material is provided with two fluid reservoirs 435 on its two sides. In this example, the two fluid reservoirs 435 are respectively arranged on the two hanging parts 41. The fluid reservoir 435 is loaded with clean water. The fluid reservoir 435 and the carrier layer 431 are manufactured in one piece, and they are interconnected by the existing one-way valve 436. When a user wants to adjust the temperature of the carrier layer 431 loaded with functional material or wants a cold compress following a hot compress, he only needs to press the fluid reservoir 435 with hand to make the clean water loaded in the fluid reservoir 435 flow to the carrier layer 431 and mix with the sodium acetate crystals, then the cooling effect would be achieved due to endothermic reaction. In use, the addition of clean water decreases the concentration of the oversaturated aqueous solution of sodium acetate, so the phase balance of the oversaturated aqueous solution of sodium acetate loaded in the carrier layer 431 is broken, when the film is used again later, the heating temperature is a little lower than before. The clean water injected into the carrier layer may accumulate at the bottom of the eye mask, which may cause an uneven temperature distribution. In order to avoid this, a little thickener (such as pectin) is added in the clean water.

In use, the user should press the stainless steel disc 4312 to cause the eye mask to release heat and apply a skincare product to the face, then apply the eye mask to the face; and after the eye mask has cooled to room temperature, he can press the fluid reservoirs 435 with hand to make the clean water loaded in the fluid reservoir 435 flow to the carrier layer 4331 and mix with the sodium acetate crystals, the process would absorb heat and refrigerate the eye mask; the refrigerated eye mask can be applied to the face again to cool the face skin. By the above processes, a cold compress following a hot compress can be available.

### Example 21

Figure 28 shows a multifunctional temperature self-regulating eye mask according to Example 21 of the present invention. In this example, the functional material loaded in the carrier layer 531 is urea (120g). The fluid reservoir 535 is loaded with clean water (120g), and the fluid reservoir 535 and the carrier layer 531 are manufactured in one piece, and they are interconnected by the existing one-way valve 536. Just as Example 20, the two fluid reservoirs 535 are respectively arranged on the hanging parts 51. When a user wants a cold compress, he only needs to press the fluid reservoirs 535 with hand to make the clean water loaded in the fluid reservoir 535 flow to the carrier layer 531 and mix with urea, then the cooling effect would be achieved due to endothermic reaction.

### Comparative Example 4

The eye mask in Comparative Example 4 is similar to that in Example 20 except that no thermal-insulation layer is provided, and it is used in the same way as described in Example 20.

The results show that the duration of the heat generation (in a heat range above 37°C) for the eye mask in Example 20 is about 35 minutes, while it is only about 20 minutes for the eye mask in Comparative Example 4; and the duration of the refrigeration for the eye mask in Example 20 is about 30 minutes, while it is only about 20 minutes for the eye mask in Comparative Example 4. In addition, it has been demonstrated in experiments that due to the thermal-insulation layer according to the invention the heat generation/refrigeration in Example 20 is significantly more homogeneous than that in Comparative Example 4, that is, the eye mask in Example 20 overcomes the problem of the eye mask in Comparative Example 4 which is either too hot or too cold for most of the time it is being used. Compared with Comparative Example 4, the eye mask in Example 20 improves the absorption effect of the skincare and the moisture content of the skin.

## Claims

1. A multifunctional temperature self-regulating film, from outside to inside comprising at least:
a carrier layer loaded with functional materials, and
a thermal-insulation layer directly contacted with the skin;
wherein the carrier layer loaded with functional materials has a sealed cavity sealed with a flexible water-impermeable material, the sealed cavity is at least loaded with a refrigerating or heat-generating chemical material; wherein the thermal-insulation layer is made of a material having waterproof and thermal-insulation properties.

2. The multifunctional temperature self-regulating film according to claim 1, wherein the thermal-insulation layer is a metallized polyethylene terephthalate film made of polyethylene terephthalate film and metal.

3. The multifunctional temperature self-regulating film according to claim 2, wherein the metal used for the metallized polyethylene terephthalate film is aluminum, nickel, chromium or a mixture thereof.

4. The multifunctional temperature self-regulating film according to claim 1, wherein the flexible water-impermeable material is made of plastics, synthetic rubbers, synthetic fibers, waterproof fabrics or waterproof papers.

5. The multifunctional temperature self-regulating film according to claim 4, wherein the flexible water-impermeable material is transparent.

6. The multifunctional temperature self-regulating film according to claim 1, wherein the sealed cavity is loaded with
a solid-liquid phase change material which generates heat when the material changes from liquid to solid, and an actuating means which is used to trigger an exothermic state of the solid-liquid phase change material.

7. The multifunctional temperature self-regulating film according to claim 6, after the liquid-to-solid phase change process of the solid-liquid phase change material has been triggered, the film maintains a heating temperature at 40°C to 55°C, and the heating time lasts 5 to 30 minutes.

8. The multifunctional temperature self-regulating film according to claim 7, wherein the solid-liquid phase change material is an oversaturated aqueous solution of sodium acetate, the actuating means is a flexible metal disc which takes a shape of an arc and has a plurality of corrugations on its surface, and the flexible metal disc is located in the oversaturated aqueous solution of sodium acetate.

9. The multifunctional temperature self-regulating film according to claim 8, wherein the oversaturated aqueous solution of sodium acetate is added with a nucleating agent.

10. The multifunctional temperature self-regulating film according to claim 9, wherein the nucleating agent is selected from one or more of the group consisting of sodium borate, sodium sulfate, sodium silicate, sodium pyrophosphate, and sodium phosphate dibasic dodecahydrate.

11. The multifunctional temperature self-regulating film according to claim 6, wherein at least one side of the carrier layer is provided with a fluid reservoir; the fluid reservoir and the carrier layer are manufactured in one piece, and they are interconnected by an existing one-way valve; the fluid reservoir is loaded with fluid, when the fluid reservoir is pressed by an external force, the fluid loaded in the fluid reservoir is injected into the carrier layer through the one-way valve to adjust the temperature of the carrier layer.

12. The multifunctional temperature self-regulating film according to claim 1, wherein the sealed cavity is loaded with a solid refrigerating chemical material which absorbs heat when the material changes from solid to liquid; at least one side of the carrier layer is provided with a fluid reservoir; the fluid reservoir and the carrier layer are manufactured in one piece, and they are interconnected by an existing one-way valve; the fluid reservoir is loaded with fluid, when the fluid reservoir is pressed by an external force, the fluid loaded in the fluid reservoir is injected into the carrier layer through the one-way valve to dissolve the solid refrigerating chemical material, such that the solid refrigerating chemical material changes from solid to liquid to absorb heat and lower the temperature of the film.

13. The multifunctional temperature self-regulating film according to claim 12, after the solid-to-liquid phase change process of the solid refrigerating chemical material has been triggered, the film maintains a cooling temperature at -3°C to 20°C, and the cooling time lasts 5 to 30 minutes.

14. The multifunctional temperature self-regulating film according to claim 12, wherein the solid refrigerating chemical material is selected from one or more of the group consisting of urea, ammonium chloride and ammonium nitrate, and the fluid loaded in the fluid reservoir is clean water; wherein the weight ratio of the solid refrigerating chemical material to the clean water is 1:1.

15. The multifunctional temperature self-regulating film according to claim 11 or 12, wherein a suitable amount of thickener is added in the fluid loaded in the fluid reservoir.

16. The multifunctional temperature self-regulating film according to claim 11 or 12, wherein the sealed cavity and/or the fluid reservoir is further loaded with a pigment.

17. The multifunctional temperature self-regulating film according to any one of claims 1 to 14, wherein the film is ergonomically designed in shape to be applied to various body parts.

18. The multifunctional temperature self-regulating film according to claim 17, wherein there is provided a layer of water-soluble hydrogel on the surface of the thermal-insulation layer that is in contact with the skin, the layer of soluble hydrogel being used for adhering the film to the skin.

19. The multifunctional temperature self-regulating film according to claim 18, wherein there is provided an active ingredient of a topically-applied healthcare product, a topically-applied medicine or a skincare product in the layer of water-soluble hydrogel.

20. The multifunctional temperature self-regulating film according to claim 11 or 12, wherein the multifunctional temperature self-regulating film is made into a face mask applied to the face, both two sides of the carrier layer loaded with functional materials are provided with a fluid reservoir which is made into a long and narrow strip, and either of the fluid reservoirs is provided with a fixture to attach the face mask to the user's head.

21. The multifunctional temperature self-regulating film according to claim 20, wherein the face mask is 2 mm to 15 mm thick.

22. A method of using the multifunctional temperature self-regulating film according to claim 20, comprising applying a skincare product or a traditional nourishing face mark to the face prior to applying the face mask.

23. A multifunctional temperature self-regulating three-dimensional face mask which has a facial concave-convex shape formed by joining two sheets together, the face portion of the three-dimensional face mask corresponding to the face from outside to inside comprising at least: a carrier layer loaded with functional materials and a thermal-insulation layer directly contacted with the skin; wherein the carrier layer loaded with functional materials has a sealed cavity sealed with a flexible water-impermeable material, the sealed cavity is at least loaded with a refrigerating or heat-generating chemical material; wherein the thermal-insulation layer is made of a material having waterproof and thermal-insulation properties.

24. The multifunctional temperature self-regulating three-dimensional face mask according to claim 23, wherein the thermal-insulation layer is a metallized polyethylene terephthalate film made of polyethylene terephthalate film and metal.

25. The multifunctional temperature self-regulating three-dimensional face mask according to claim 24, wherein the metal used for the metallized polyethylene terephthalate film is aluminum, nickel, chromium or a mixture thereof.

26. The multifunctional temperature self-regulating three-dimensional face mask according to claim 23, wherein the flexible water-impermeable material is made of plastics, synthetic rubbers, synthetic fibers, waterproof fabrics or waterproof papers.

27. The multifunctional temperature self-regulating three-dimensional face mask according to claim 26, wherein the flexible water-impermeable material is transparent.

28. The multifunctional temperature self-regulating three-dimensional face mask according to claim 23, wherein the sealed cavity is loaded with a solid-liquid phase change material which generates heat when the material changes from liquid to solid, and an actuating means which is used to trigger an exothermic state of the solid-liquid phase change material.

29. The multifunctional temperature self-regulating three-dimensional face mask according to claim 28, after the liquid-to-solid phase change process of the solid-liquid phase change material has been triggered, the film maintains a heating temperature at 40°C to 55°C, and the heating time lasts 5 to 30 minutes.

30. The multifunctional temperature self-regulating three-dimensional face mask according to claim 29, wherein the solid-liquid phase change material is an oversaturated aqueous solution of sodium acetate, and the actuating means is a flexible metal disc which takes a shape of an arc and has a plurality of corrugations on its surface, and the flexible metal disc is located in the oversaturated aqueous solution of sodium acetate.

31. The multifunctional temperature self-regulating three-dimensional face mask according to claim 30, wherein the oversaturated aqueous solution of sodium acetate is added with a nucleating agent.

32. The multifunctional temperature self-regulating three-dimensional face mask according to claim 31, wherein the nucleating agent is selected from one or more of the group consisting of sodium borate, sodium sulfate, sodium silicate, sodium pyrophosphate and sodium phosphate dibasic dodecahydrate.

33. The multifunctional temperature self-regulating three-dimensional face mask according to claim 28, wherein at least one side of the carrier layer is provided with a fluid reservoir; wherein the fluid reservoir and the carrier layer are manufactured in one piece, and they are interconnected by an existing one-way valve, and the fluid reservoir is loaded with fluid; when the fluid reservoirs is pressed by an external force, the fluid loaded in the fluid reservoir is injected into the carrier layer through the one-way valve to adjust the temperature of the carrier layer.

34. The multifunctional temperature self-regulating three-dimensional face mask according to claim 23, wherein the sealed cavity is loaded with a solid refrigerating chemical material which absorbs heat when the material changes from solid to liquid; at least one side of the carrier layer is provided with a fluid reservoir; the fluid reservoir and the carrier layer are manufactured in one piece, and they are interconnected by an existing one-way valve; the fluid reservoir is loaded with fluid, when the fluid reservoir is pressed by an external force, the fluid loaded in the fluid reservoir is injected into the carrier layer through the one-way valve to dissolve the solid refrigerating chemical material, such that the solid refrigerating chemical material changes from solid to liquid to absorb heat and lower the temperature of the film.

35. The multifunctional temperature self-regulating three-dimensional face mask according to claim 34, after the solid-to-liquid phase change process of the solid refrigerating chemical material has been triggered, the film maintains a cooling temperature at -3°C to 20°C, and the cooling time lasts 5 to 30 minutes.

36. The multifunctional temperature self-regulating three-dimensional face mask according to claim 34, wherein the solid refrigerating chemical material is selected from one or more of the group consisting of urea, ammonium chloride, ammonium nitrate, and the fluid loaded in the fluid reservoir is clean water; wherein the weight ratio of the solid refrigerating chemical material to the clean water is 1:1.

37. The multifunctional temperature self-regulating three-dimensional face mask according to claim 33 or 34, wherein a a suitable amount of thickener is added in the fluid loaded in the fluid reservoir.

38. The multifunctional temperature self-regulating three-dimensional face mask according to claim 33 or 34, wherein the sealed cavity and/or the fluid reservoir is further loaded with a pigment.

39. The multifunctional temperature self-regulating three-dimensional face mask according to claim 33 or 34, the three-dimensional face mask is 2mm to 15mm thick.

40. The multifunctional temperature self-regulating three-dimensional face mask according to claim 23, wherein there is provided a layer of water-soluble hydrogel on the surface of the thermal-insulation layer that is in contact with the skin, the layer of soluble hydrogel being used for adhering the face mask to the skin.

41. The multifunctional temperature self-regulating three-dimensional face mask according to claim 40, wherein there is provided an active ingredient of a topically-applied healthcare product, a topically-applied medicine or a skincare product in the layer of water-soluble hydrogel.

42. The multifunctional temperature self-regulating three-dimensional face mask according to claim 23, wherein the three-dimensional face mask comprises a left side sheet and a right side sheet respectively matching the left area of the face and the right area of the face, front edge portions of the left side sheet and the right side sheet are provided with an adhesive material, the left side sheet and the right side sheet are thereby adhered together by the adhesive material along a median line of the face to form a facial concave-convex shape.

43. The multifunctional temperature self-regulating three-dimensional face mask according to claim 42, wherein either of the front edge portions of the left side sheet and the right side sheet has a protrusion projecting outwards from above down and matching the shape of the left area or right area of the external nose, a mouth portion which is a notch matching the mouth is provided below the protrusion, a portion of the front edge portion below the notch has an arc shape with a certain radian and extends downwards to the bottom the face mask; except the notch matching the mouth, either of the front edge portions of the left side sheet and the right side sheet is provided with an adhesive material, and the left side sheet and the right side sheet are thereby adhered together by the adhesive material.

44. The multifunctional temperature self-regulating three-dimensional face mask according to claim 43, wherein a glabella portion of either of the front edge portions of the left side sheet and the right side sheet which faces a space between the user's eyebrows, just above the protrusion, is formed as an arc which is slightly concaved inwards.

45. The multifunctional temperature self-regulating three-dimensional face mask according to claim 43, wherein an eye-and-glabella portion of either of the front edge portions of the left side sheet and the right side sheet which faces the user's eyes and glabella, just above the protrusion, is formed as a notch which is concaved inwards; except the eye-and-glabella portion and the mouth portion, either of the front edge portions of the left side sheet and the right side sheet are provided with an adhesive material , and the left side sheet and the right side sheet are thereby adhered together by the adhesive material.

46. The multifunctional temperature self-regulating three-dimensional face mask according to any one of claims 42-45, there is a fixing strap provided on either of the rear edge portions of the left side sheet and the right side sheet, and there is a velcro provided at the end of the fixing strap.

47. A method of using the multifunctional temperature self-regulating three-dimensional face mask according to claim 46, comprising first applying a skincare product or a traditional nourishing face mark to the face prior to applying the three-dimensional face mask; or, pre-coating a skincare product on the surface of the thermal-insulation layer that is in contact with the skin, joining the left side sheet and the right side sheet together, then applying the joined three-dimensional face mask to the face.

48. A multifunctional temperature self-regulating face mask, the face portion of the face mask corresponding to the face from outside to inside comprising at least: a carrier layer loaded with functional materials and a thermal-insulation layer directly contacted with the skin; wherein the carrier layer loaded with functional materials has a sealed cavity sealed with a flexible water-impermeable material, the sealed cavity is at least loaded with a refrigerating or heat-generating chemical material; wherein the thermal-insulation layer is made of a material having waterproof and thermal-insulation properties.

49. The multifunctional temperature self-regulating face mask according to claim 48, wherein the thermal-insulation layer is a metallized polyethylene terephthalate film made of polyethylene terephthalate film and metal.

50. The multifunctional temperature self-regulating face mask according to claim 49, wherein the metal used for the metallized polyethylene terephthalate film is aluminum, nickel, chromium or a mixture thereof.

51. The multifunctional temperature self-regulating face mask according to claim 48, wherein the flexible water-impermeable material is made of plastics, synthetic rubbers, synthetic fibers, waterproof fabrics or waterproof papers.

52. The multifunctional temperature self-regulating face mask according to claim 52, wherein the flexible water-impermeable material is transparent.

53. The multifunctional temperature self-regulating face mask according to claim 48, wherein the sealed cavity is loaded with a solid-liquid phase change material which generates heat when the material changes from liquid to solid, and an actuating means which is used to trigger an exothermic state of the solid-liquid phase change material.

54. The multifunctional temperature self-regulating face mask according to claim 53, after the liquid-to-solid phase change process of the solid-liquid phase change material has been triggered, the film maintains a heating temperature at 40°C to 55°C, and the heating time lasts 5 to 30 minutes.

55. The multifunctional temperature self-regulating face mask according to claim 54, wherein the solid-liquid phase change material is an oversaturated aqueous solution of sodium acetate, and the actuating means is a flexible metal disc which takes a shape of an arc and has a plurality of corrugations on its surface, and the flexible metal disc is located in the oversaturated aqueous solution of sodium acetate.

56. The multifunctional temperature self-regulating face mask according to claim 55, wherein the oversaturated aqueous solution of sodium acetate is added with a nucleating agent.

57. The multifunctional temperature self-regulating face mask according to claim 56, wherein the nucleating agent is selected from one or more of the group consisting of sodium borate, sodium sulfate, sodium silicate, sodium pyrophosphate and sodium phosphate dibasic dodecahydrate.

58. The multifunctional temperature self-regulating face mask according to claim 53, wherein at least one side of the carrier layer is provided with a fluid reservoir; wherein the fluid reservoir and the carrier layer are manufactured in one piece, and they are interconnected by an existing one-way valve, and the fluid reservoir is loaded with fluid; when the fluid reservoirs is pressed by an external force, the fluid loaded in the fluid reservoir is injected into the carrier layer through the one-way valve to adjust the temperature of the carrier layer.

59. The multifunctional temperature self-regulating face mask according to claim 48, wherein the sealed cavity is loaded with a solid refrigerating chemical material which absorbs heat when the material changes from solid to liquid; at least one side of the carrier layer is provided with a fluid reservoir; the fluid reservoir and the carrier layer are manufactured in one piece, and they are interconnected by an existing one-way valve; the fluid reservoir is loaded with fluid, when the fluid reservoir is pressed by an external force, the fluid loaded in the fluid reservoir is injected into the carrier layer through the one-way valve to dissolve the solid refrigerating chemical material, such that the solid refrigerating chemical material changes from solid to liquid to absorb heat and lower the temperature of the film.

60. The multifunctional temperature self-regulating face mask according to claim 59, after the solid-to-liquid phase change process of the solid refrigerating chemical material has been triggered, the film maintains a cooling temperature at -3°C to 20°C, and the cooling time lasts 5 to 30 minutes.

61. The multifunctional temperature self-regulating face mask according to claim 59, wherein the solid refrigerating chemical material is selected from one or more of the group consisting of urea, ammonium chloride, ammonium nitrate, and the fluid loaded in the fluid reservoir is clean water; wherein the weight ratio of the solid refrigerating chemical material to the clean water is 1:1.

62. The multifunctional temperature self-regulating face mask according to claim 58 or 59, wherein a a suitable amount of thickener is added in the fluid loaded in the fluid reservoir.

63. The multifunctional temperature self-regulating face mask according to claim 58 or 59, wherein the sealed cavity and/or the fluid reservoir is further loaded with a pigment.

64. The multifunctional temperature self-regulating face mask according to claim 58 or 59, the face mask is 2mm to 15mm thick.

65. The multifunctional temperature self-regulating face mask according to claim 48, wherein there is provided a layer of water-soluble hydrogel on the surface of the thermal-insulation layer that is in contact with the skin, the layer of soluble hydrogel being used for adhering the film to the skin.

66. The multifunctional temperature self-regulating face mask according to claim 65, wherein there is provided an active ingredient of a topically-applied healthcare product, a topically-applied medicine or a skincare product in the layer of water-soluble hydrogel.

67. The multifunctional temperature self-regulating face mask according to claim 48, wherein the face mask has notches matching the user's nose and mouth, respectively.

68. The multifunctional temperature self-regulating face mask according to claim 67, wherein the face mask also has notches matching the user's eyes.

69. The multifunctional temperature self-regulating face mask according to claim 68, wherein there is a fixing strap provided on either side of the face mask, and there is a velcro provided at the end of the fixing strap.

70. A method of using the multifunctional temperature self-regulating face mask according to claim 69, comprising: applying a skincare product or a traditional nourishing face mark to the face prior to applying the multifunctional temperature self-regulating face mask; or, pre-coating a skincare product on the surface of the thermal-insulation layer that is in contact with the skin, then applying the pre-coated multifunctional temperature self-regulating face mask to the face.

71. A multifunctional temperature self-regulating eye mask, the eye portion of the eye mask corresponding to the eye from outside to inside comprising at least: a carrier layer loaded with functional materials and a thermal-insulation layer directly contacted with the skin; wherein the carrier layer loaded with functional materials has a sealed cavity sealed with a flexible water-impermeable material, the sealed cavity is at least loaded with a refrigerating or heat-generating chemical material; wherein the thermal-insulation layer is made of a material having waterproof and thermal-insulation properties.

72. The multifunctional temperature self-regulating eye mask according to claim 71, wherein the thermal-insulation layer is a metallized polyethylene terephthalate film made of polyethylene terephthalate film and metal.

73. The multifunctional temperature self-regulating eye mask according to claim 72, wherein the metal used for the metallized polyethylene terephthalate film is aluminum, nickel, chromium or a mixture thereof.

74. The multifunctional temperature self-regulating eye mask according to claim 71, wherein the flexible water-impermeable material is made of plastics, synthetic rubbers, synthetic fibers, waterproof fabrics or waterproof papers.

75. The multifunctional temperature self-regulating eye mask according to claim 74, wherein the flexible water-impermeable material is transparent.

76. The multifunctional temperature self-regulating eye mask according to claim 71, wherein the sealed cavity is loaded with a solid-liquid phase change material which generates heat when the material changes from liquid to solid, and an actuating means which is used to trigger an exothermic state of the solid-liquid phase change material.

77. The multifunctional temperature self-regulating eye mask according to claim 76, after the liquid-to-solid phase change process of the solid-liquid phase change material has been triggered, the film maintains a heating temperature at 40 to 55°C, and the heating time lasts 5 to30 minutes.

78. The multifunctional temperature self-regulating eye mask according to claim 77, wherein the solid-liquid phase change material is an oversaturated aqueous solution of sodium acetate, and the actuating means is a flexible metal disc which takes a shape of an arc and has a plurality of corrugations on its surface, and the flexible metal disc is located in the oversaturated aqueous solution of sodium acetate.

79. The multifunctional temperature self-regulating eye mask according to claim 78, wherein the oversaturated aqueous solution of sodium acetate is added with nucleating agent.

80. The multifunctional temperature self-regulating eye mask according to claim 79, wherein the nucleating agent is selected from one or more of the group consisting of sodium borate, sodium sulfate, sodium silicate, sodium pyrophosphate and sodium phosphate dibasic dodecahydrate.

81. The multifunctional temperature self-regulating eye mask according to claim 76, wherein at least one side of the carrier layer is provided with a fluid reservoir; wherein the fluid reservoir and the carrier layer are manufactured in one piece, and they are interconnected by an existing one-way valve, and the fluid reservoir is loaded with fluid; when the fluid reservoirs is pressed by an external force, the fluid loaded in the fluid reservoir is injected into the carrier layer through the one-way valve to adjust the temperature of the carrier layer.

82. The multifunctional temperature self-regulating eye mask according to claim 71, wherein the sealed cavity is loaded with a solid refrigerating chemical material which absorbs heat when the material changes from solid to liquid; at least one side of the carrier layer is provided with a fluid reservoir; the fluid reservoir and the carrier layer are manufactured in one piece, and they are interconnected by an existing one-way valve; the fluid reservoir is loaded with fluid, when the fluid reservoir is pressed by an external force, the fluid loaded in the fluid reservoir is injected into the carrier layer through the one-way valve to dissolve the solid refrigerating chemical material, such that the solid refrigerating chemical material changes from solid to liquid to absorb heat and lower the temperature of the film.

83. The multifunctional temperature self-regulating eye mask according to claim 82, after the solid-to-liquid phase change process of the solid refrigerating chemical material has been triggered, the film maintains a cooling temperature at -3 to 20°C, and the cooling time lasts 5 to 30 minutes.

84. The multifunctional temperature self-regulating eye mask according to claim 82, wherein the solid refrigerating chemical material is selected from one or more of the group consisting of urea, ammonium chloride, ammonium nitrate, and the fluid loaded in the fluid reservoir is clean water; wherein the weight ratio of the solid refrigerating chemical material to the clean water is 1:1.

85. The multifunctional temperature self-regulating eye mask according to claim 81 or 82, wherein a suitable amount of thickener is added in the fluid loaded in the fluid reservoir.

86. The multifunctional temperature self-regulating eye mask according to claim 81 or 82, wherein the sealed cavity and/or the fluid reservoir is further loaded with a pigment.

87. The multifunctional temperature self-regulating eye mask according to claim 81 or 82, the eye mask is 2mm to 15mm thick.

88. The multifunctional temperature self-regulating eye mask according to claim 71, wherein there is provided a layer of water-soluble hydrogel on the surface of the thermal-insulation layer that is in contact with the skin, the layer of soluble hydrogel being used for adhering the film to the skin.

89. The multifunctional temperature self-regulating eye mask according to claim 88, wherein there is provided an active ingredient of a topically-applied healthcare product, a topically-applied medicine or a skincare product in the layer of water-soluble hydrogel.

90. The multifunctional temperature self-regulating eye mask according to claim 71, wherein the eye mask comprises hanging parts which have ears slits and can be hung on the user's ears.

91. The multifunctional temperature self-regulating eye mask according to claim 90, wherein a slit is vertically cut along a lower half of a central vertical axis of the eye mask.
